# EUROPEAN PATENT APPLICATION

(11) **EP 2 954 888 A1**
(43) Date of publication of application: **16.12.2015**
(21) Application number: 15172604.9
(22) Date of filing: 31.08.2011
(51) Int. Cl.: A61K 9/14, A61K 31/58, A61P 11/06, A61K 31/137, A61K 31/133

(54) **DRY POWDER INHALATION DRUG PRODUCTS EXHIBITING MOISTURE CONTROL PROPERTIES AND METHODS OF ADMINISTERING THE SAME**

(30) Priority: 31.08.2010 US 378412 P
(62) Divisional of application: 11755043.4
(71) Applicant: GlaxoSmithKline Intellectual Property Development Limited, Brentford Middlesex TW8 9GS (GB)
(72) Inventor: ASWANIA, Osama, Stevenage, Hertfordshire SG1 2NY (GB); JIANG, Zhong, Ware, Hertfordshire SG12 0DP (GB); ROCHE, Trevor Charles, Ware, Hertfordshire SG12 0DP (GB); WHITAKER, Mark, Ware, Hertfordshire SG12 0DP (GB)
(74) Representative: Florence, Julia Anne

(57) **Abstract**

A drug product comprising:
a dry powder inhalation device containing one or more pharmaceutical compositions present therein, wherein the one or more pharmaceutical compositions comprise active ingredients (I) 4-{(1*R*)-2-[(6-{2-[(2,6-dichlorobenzyl)oxy]ethoxy}hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof;
a hygroscopic material; and
a package which encompasses the dry powder inhalation device and the hygroscopic material defining an enclosed volume therein;
wherein each of the active ingredients (I) and (II) are present in the same or different pharmaceutical compositions, and wherein the enclosed volume within the package exhibits a Relative Humidity of from 20% to 40%.

## Description

### CROSS-REFERENCE TO RELATED APPLICATIONS

This application references U.S. Provisional Application Serial No. 61/378,409 entitled "Dry Powder Inhalation Drug Products Exhibiting Moisture Control Properties and Methods of Administering the Same" (Attorney Docket No. PR64313) filed August 31, 2010, the disclosure of which is incorporated by reference herein in their entirety.

The present application claims priority to U.S. Provisional Application Serial No. 61/378,412 filed August 31, 2010, the disclosure of which is incorporated herein by reference in its entirety.

### FIELD OF THE INVENTION

The invention generally relates to inhalation drug products and methods of manufacturing the same.

### BACKGROUND OF THE INVENTION

Inhalers are hand-held portable devices that deliver medication directly to the lungs. One class of inhalers is passive dry powder inhalers ("DPI"). A passive DPI is a patient driven device wherein the action of breathing in through the device draws the powder formulation into the respiratory tract. DPI is well recognized as a method of drug delivery to the lung for treatment of pulmonary and systemic diseases.

A DPI formulation is generally a powder blend of active ingredients and a bulk solid diluent, such as lactose. The inhaled particle size of the active ingredients should be optimized to deliver the drug deep into the lung to achieve efficacy. This efficacious particle size, or fine particle mass ("FPM"), typically lies between 1-5 µm whereas particles larger than this, 5-10 µm, tend to be deposited in the upper airways without reaching the site of action and the very smallest particles, or very fine particle mass ("vFPM"), <1 µm, can resultantly be exhaled therefore typically not achieving the desired therapeutic levels. The Food and Drug Administration has recognized that the FPM of particles in a DPI device can be affected by environmental conditions, humidity in particular, and has suggested that manufacturers of such devices assess the effect of different environmental conditions on various interactive forces within the DPI device, which together influence the fluidization and aerosolization behavior of the formulation and, hence, performance (see FDA Guidance, Metered Dose Inhaler (MDI) and Dry Powder Inhaler (DPI) Drug Products Chemistry, Manufacturing, and Controls Documentation).

It is desirable to control the humidity within a DPI device, and hence the FPM. One approach to this involves the use of a desiccant system within the DPI such as shown in publication WO2008040841 (Lab Pharma Ltd., filed September 12, 2007). This approach uses a desiccant system comprising an air-tight container containing a dry desiccant, and a drug chamber containing the inhalation powder where the air-tight container is arranged inside the drug chamber or in its vicinity with the desiccant being capable of maintaining a fixed point of humidity as a saturated solution of at least one salt. The system is intended to maintain the maximum relative humidity around the powder formulation within a specified range for a prolonged period. Similarly, the reservoir based multi dose dry powder inhalers (e.g., Turbuhaler^{®} made commercially available by Astra Zeneca of Wilmington, Delaware (see e.g., Wetterlim (Pharm. Res 5, 506-508, 1988)) contain a desiccant store in such inhalers.

An alternative approach to controlling the moisture absorption by dry powder products is shown in publication US20080063719 (Vectura Limited, filed April 29, 2005). This approach shows an inhalable dry powder formulation of glycopyrrolate with a stability of at least 1 year under normal conditions by storing in packaging made from a material which itself has a moisture content less than 10%, preferably less than 5% and more preferably less than 3%. Glycopyrrolate has been found to have an acute problem with respect to stability, especially immediately following conventional micronisation process. Individual capsule doses of glycopyrrolate for a suitable dispensing technique are used with the capsules being made of hypromellose, plasticized gelatin, starch, chitosan, synthetic plastic or thermoplastics. These materials were selected as capsules as they can maintain the glycopyrrolate with a suitable range of moisture for aerosol delivery.

Notwithstanding any potential progress that has been made regarding controlling the humidity within the DPI device, the range of acceptable humidity for specific actives and blends is not well understood and is difficult, if not impossible, to predict.

### SUMMARY OF THE INVENTION

In a first aspect, the invention provides a drug product. The drug product comprises a dry powder inhalation device containing one or more pharmaceutical compositions therein, wherein the one or more pharmaceutical compositions comprise active ingredients (I)) 4-{(1*R*)-2-[(6-{2-[(2,6-dichlorobenzyl)oxy]ethoxy} hexyl)amino]-1-hydroxyethyl}-2-(hydroxymethyl)phenol, or a salt thereof and (II) (6α, 11ß, 16α, 17 α)-6,9-difluoro-17-{[(fluoromethyl)thio]carbonyl}-11-hydroxy-16-methyl-3-oxoandrosta-1,4-dien-17-yl 2- furancarboxylate or a solvate thereof; a hygroscopic material, a package which encompasses the dry powder inhalation device and hygroscopic material defining an enclosed volume therein, wherein each of the active ingredients (I) and (II) are present in the same or different pharmaceutical compositions, and wherein the enclosed volume within the package exhibits a Relative Humidity of from 20% to 40%.

By virtue of the invention, more particularly by judicious selection of Relative Humidity values, and as set forth in greater detail herein, advantageously the drug product is capable of exhibiting improved shelf life and more stable fine particle mass as a result of the Relative Humidity with the package enclosed volume being controlled within a specified range.

In another aspect, the invention provides a method of treating a respiratory disorder. The method comprises administering to a patient by oral inhalation (I) 4-{(1*R*)-2-[(6-{2-[(2,6-dichlorobenzyl)oxy]ethoxy}hexyl)amino]-1-hydroxyethyl}-2-(hydroxymethyl)phenol, or a salt thereof and (II) (6α,11ß,16α,17 α)-6,9-difluoro-17-{[(fluoromethyl)thio]carbonyl}-11-hydroxy-16-methyl-3-oxoandrosta-1,4-dien-17-yl 2- furancarboxylate or a solvate thereof using a drug product as described herein in the first aspect.

In another aspect, the invention provides a process of producing a drug product comprising subjecting a hygroscopic material to moisture exposure sufficient to attain a predetermined relative humidity; combining the hygroscopic material with an inhalation device containing one or more pharmaceutical compositions therein, wherein the one or more pharmaceutical compositions comprise active ingredients (I) 4-{(1*R*)-2-[(6-{2-[(2,6-dichlorobenzyl)oxy]ethoxy} hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; wherein each of the active ingredients (I) and (II) are present in the same or different pharmaceutical compositions, and thereafter enclosing the dry powder inhalation device and hygroscopic material within a package to define an enclosed volume therein forming a drug product and wherein the hydration level of the hygroscopic material is such that enclosed volume has a Relative Humidity of from 20% to 40%. The subjecting and combining steps may occur together or separately.

These and other aspects are provided by the invention as described herein.

### BRIEF DESCRIPTION OF THE FIGURES

Figure 1 depicts the % Nominal fine particle mass as a function of time for compound B in an inhaled formulation.
Figure 2 depicts the % Nominal fine particle mass as a function of time for compound A in an inhaled formulation.
Figure 3 depicts the % Nominal fine particle mass as a function of time for compound B in an inhaled formulation.
Figure 4 depicts the % Nominal fine particle mass as a function of time for compound A in an inhaled formulation.
Figure 5 depicts the % Nominal fine particle mass as a function of time for compound B in an inhaled formulation.
Figure 6 depicts the % Nominal fine particle mass as a function of time for compound A in an inhaled formulation.
Figure 7 depicts the % Nominal fine particle mass as a function of time for compound B in an inhaled formulation.
Figure 8 depicts the % Nominal fine particle mass as a function of time for compound B in an inhaled formulation.
Figure 9 depicts the % Nominal fine particle mass as a function of time for compound A in an inhaled formulation.
Figure 10 depicts the % Nominal fine particle mass as a function of time for compound A in an inhaled formulation.
Figure 11 depicts the % Nominal fine particle mass as a function of time for compound B in an inhaled formulation.
Figure 12 depicts the % Nominal fine particle mass as a function of time for compound A in an inhaled formulation.
Figure 13 depicts the % Nominal fine particle mass as a function of time for compound B in an inhaled formulation.
Figure 14 depicts the % Nominal fine particle mass as a function of time for compound A in an inhaled formulation.
Figure 15 depicts the % Nominal fine particle mass as a function of time for compound B in an inhaled formulation.
Figure 16 depicts the % Nominal fine particle mass as a function of time for compound A in an inhaled formulation.
Figure 17 depicts the mass median aerodynamic diameter as a function of time for compound B in an inhaled formulation.
Figure 18 depicts the mass median aerodynamic diameter as a function of time for compound B in an inhaled formulation.
Figure 19 depicts the mass median aerodynamic diameter as a function of time for compound A in an inhaled formulation.
Figure 20 depicts the mass median aerodynamic diameter as a function of time for compound A in an inhaled formulation.
Figure 21 depicts the mass median aerodynamic diameter as a function of time for compound B in an inhaled formulation.
Figure 22 depicts the mass median aerodynamic diameter as a function of time for compound A in an inhaled formulation.
Figure 23 depicts the mass median aerodynamic diameter as a function of time for compound B in an inhaled formulation.
Figure 24 depicts the mass median aerodynamic diameter as a function of time for compound A in an inhaled formulation.
Figure 25 depicts the mass median aerodynamic diameter as a function of time for compound B in an inhaled formulation.
Figure 26 depicts the mass median aerodynamic diameter as a function of time for compound A in an inhaled formulation.
Figure 27 depicts the geometric standard deviation as a function of time for compound B in an inhaled formulation.
Figure 28 depicts the geometric standard deviation as a function of time for compound B in an inhaled formulation.
Figure 29 depicts the geometric standard deviation as a function of time for compound A in an inhaled formulation.
Figure 30 depicts the geometric standard deviation as a function of time for compound A in an inhaled formulation.
Figure 31 depicts the mass geometric standard deviation as a function of time for compound B in an inhaled formulation.
Figure 32 depicts the mass geometric standard deviation as a function of time for compound B in an inhaled formulation.
Figure 33 depicts the mass geometric standard deviation as a function of time for compound B in an inhaled formulation.
Figure 34 depicts the geometric standard deviation as a function of time for compound A in an inhaled formulation.
Figure 35 depicts the geometric standard deviation as a function of time for compound A in an inhaled formulation.
Figure 36 depicts the geometric standard deviation as a function of time for compound A in an inhaled formulation.
Figure 37 depicts the pack %RH as a function of time for the 50/25 µg compound A/B respectively in an inhaled formulation.
Figure 38 depicts the pack %RH as a function of time for the 100/25 µg compound A/B respectively in an inhaled formulation.
Figure 39 depicts the pack %RH as a function of time for the 200/25 µg compound A/B respectively in an inhaled formulation.
Figure 40 represents an exploded view of an embodiment of a drug product in accordance with the invention.
Figure 41 represents an embodiment of a drug product in accordance with the invention.

### DETAILED DESCRIPTION OF THE INVENTION

The invention will now be described with respect to the embodiments presented herein. Before describing the present invention in detail, it is to be understood that this invention is not limited to particularly exemplified structures, apparatus, systems, materials or methods as such may, of course, vary. Thus, although a number of apparatus, systems and methods similar or equivalent to those described herein can be used in the practice of the present invention, the preferred apparatus, systems and methods are described herein.

It is also to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

Further, all publications, patents and patent applications cited herein, whether supra or infra, are hereby incorporated by reference in their entirety.

Finally, as used in this specification and the appended claims, the singular forms "a", "an", "the" and "one" include plural referents unless the content clearly dictates otherwise. Thus, for example, reference to "a salt" includes two or more such salts; reference to "a constituent" includes two or more such constituents and the like.

In one aspect, the invention provides a drug product. The drug product comprises a dry powder inhalation device containing one or more pharmaceutical compositions therein (e.g., one or two), wherein the one or more pharmaceutical compositions comprise active ingredients (I) 4-{(1*R*)-2-[(6-{2-[(2,6-dichlorobenzyl)oxy]ethoxy}hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; a hygroscopic material, and a package which encompasses the dry powder inhalation device and hygroscopic material wherein each of the active ingredients (I) and (II) are present in the same or different pharmaceutical compositions, and wherein the enclosed volume within the package exhibits a Relative Humidity of from 20% to 40%. In another aspect, the invention provides a drug product comprising a dry powder inhalation device containing two pharmaceutical compositions present therein, wherein one of the two pharmaceutical compositions comprise active ingredient (I) 4-{(1*R*)-2-[(6-{2-[(2,6-dichlorobenzyl)oxy]ethoxy}hexyl)amino]-1-hydroxyethyl}-2-(hydroxymethyl)phenol, or a salt thereof, and the other of the two pharmaceutical compositions comprise active ingredient (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; a hygroscopic material, and a package which encompasses the dry powder inhalation device and hygroscopic material wherein and wherein the enclosed volume within the package exhibits a Relative Humidity of from 20% to 40%. As alluded to herein below, the two pharmaceutical compositions may be present in discrete containers (e.g., two or more containers).

Notwithstanding the above range, in various embodiments, the Relative Humidity in the enclosed volume may range from, at a lower end, any of the values, 15, 16, 17, 18 19, 20, 21, 22, 23, 24, 25, 26, 27, 28, 29, 30, 31, 32, 33, 34, 35, 36, 37, 38, 39 or 40 % RH to, at an upper end, any of the values, 17, 18, 19, 20, 21, 22, 23, 24, 25, 26, 27, 28, 29, 30, 31, 32, 33, 34, 35, 36, 37, 38, 39, or 40%RH. In addition to ranges above, the invention encompasses all singular values mentioned above; i.e., the Relative Humidity may be 15%, 16%, 17%, 18%, 19%, 20% etc, through 40%. RH.

Such relative humidity is obtained by applying suitable conditions to appropriate structures as described herein.

For the purposes of the invention, the term "pharmaceutical composition" may encompass pharmaceutical formulations and in particular, dry powder pharmaceutical formulations suitable for administration via inhalation.

The term "hygroscopic material" may encompass, without limitation, desiccants and humectants. Used herein, a hygroscopic material is one having the property of readily imbibing moisture from the atmosphere, while a desiccant is a drying agent, a substance which absorbs moisture and a humectant is any substance that is added to another substance to keep it moist.

In various embodiments, the one or more pharmaceutical compositions may comprise the compounds (I) and (II). In various embodiments, the one or more pharmaceutical compositions may consist essentially of the compounds (I) and (II), i.e., exclude other active ingredients or medicaments. In various embodiments, the one or more pharmaceutical compositions may consist of the compounds (I) and (II) and at least one inert ingredient, as described herein, or may be free of inert ingredients.

The term "drug product" is to be construed to encompass a dry powder inhalation device, hygroscopic material and a package which encloses the dry powder inhalation device and the hygroscopic material. In a preferred embodiment, the package is a low-moisture-permeable-package. As an example, the term 'low-moisture-permeable-package" may be defined to have a low moisture vapor transmission rate (MVTR) that is believed to be dependent on the amount of hygroscopic material (e.g., desiccant) used in the drug product. As an example, assuming that 8 gms. as a maximum amount of hygroscopic material is used, then the MVTR should not exceed 2 mg/day/drug product, measure according to standard technique.

Hygroscopic materials, which encompass, for example, desiccants and humectants, can be pre- or partially hydrated to reach a predetermined level of moisture absorption or adsorption. These techniques are typically based on weight gain wherein the dry hygroscopic material is brought up to a percent absorbed or adsorbed moisture designed to produce the desired %RH within the final product. This process of pre- or partial hydration can be accomplished via various embodiments. One embodiment would be exposing the desiccant to a predetermined temperature and humidity environment based on anticipated storage conditions, for example, 25°C and 20%RH for a period up to the equilibrium point i.e. the time when weight gain due to moisture absorption stops increasing. Another embodiment would be an accelerated hydration process in which a chamber with higher than normal humidity level is used to shorten the time of exposure to pre-hydrate the desiccant material, for example, 25°C and 80 %RH for a 12 to 18 hour period.

Another way to reach the same predetermined moisture content level is by saturating the hygroscopic material first with water and then controlled drying it to the desired level of moisture content designed to produce the desired %RH within the final product. Any of the hydration techniques can be accomplished with vapor or liquid water.

An inhalation device present in a drug product contains a compound from group (I), i.e., is 4-{(1*R*)-2-[(6-{2-[(2,6-dichlorobenzyl)oxy]ethoxy}hexyl)amino]-1-hydroxyethyl}-2-(hydroxymethyl)phenol, as well as salts thereof. These compounds are described in U.S. Patent Nos. 7,361,787 and 7,439,393, along with methods of making the same. Such a compound is a beta.₂-adrenoreceptor agonist suitable, as an example, for once-daily administration. Salts of this compound which are suitable for use in medicine are those wherein the counterion is pharmaceutically acceptable, and are also within the scope of the invention.

The compound of (I) may be represented by the formula:

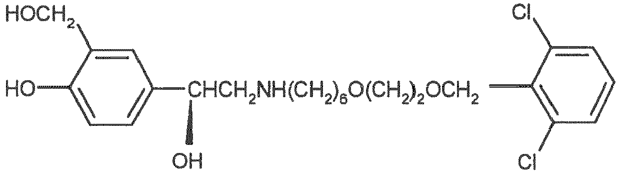

Suitable salts that may be formed include those formed with both organic and inorganic acids. Such salts include, without limitation, those which are pharmaceutically acceptable. Pharmaceutically acceptable acid addition salts include those formed from hydrochloric, hydrobromic, sulphuric, citric, tartaric, phosphoric, lactic, pyruvic, acetic, trifluoroacetic, triphenylacetic, phenylacetic, substituted phenyl acetic eg. methoxyphenyl acetic, sulphamic, sulphanilic, succinic, oxalic, fumaric, maleic, malic, glutamic, aspartic, oxaloacetic, methanesulphonic, ethanesulphonic, arylsulponic (for example p-toluenesulphonic, benzenesulphonic, naphthalenesulphonic or naphthalenedisulphonic), salicylic, glutaric, gluconic, tricarballylic, mandelic, cinnamic, substituted cinnamic (for example, methyl, methoxy, halo or phenyl substituted cinnamic, including 4-methyl and 4-methoxycinnamic acid and α-phenyl cinnamic acid), ascorbic, oleic, naphthoic, hydroxynaphthoic (for example 1- or 3-hydroxy-2-naphthoic), naphthaleneacrylic (for example naphthalene-2-acrylic), benzoic, 4-methoxybenzoic, 2- or 4-hydroxybenzoic, 4-chlorobenzoic, 4-phenylbenzoic, bezeneacrylic (for example 1,4-benzenediacrylic) and isethionic acids. Pharmaceutically acceptable base salts include ammonium salts, alkali metal salts such as those of sodium and potassium, alkaline earth metal salts such as those of calcium and magnesium and salts with organic bases such as dicyclohexyl amine and N-methyl-D-glucamine.

A preferred compound from group (I) is the salt 4-{(1*R*)-2-[(6-{2-[(2,6-dichlorobenzyl)oxy]ethoxy}hexyl)amino]-1-hydroxyethyl}-2-(hydroxymethyl)phenol triphenylacetate. ("Compound B"). Compound B is known as vilanterol trifenatate.

The other compound delivered from the device is selected from the group (II), i.e(6α,11ß,16α,17ß)-6,9-difluoro-17-{[(fluoromethyl)thio]carbonyl}-11-hydroxy-16-methyl-3-oxoandrosta-1,4-dien-17-yl 2- furancarboxylate or a solvate thereof, including those that are pharmaceutically acceptable. Such compounds and methods of making the same are set forth in U.S. Patent No. 7,101,866. Such compounds are anti-inflammatory compounds of the androstane series. Solvates of this compound which are suitable for use in medicine are those wherein the counterion or associated solvent is pharmaceutically acceptable, and are also within the scope of the invention. However, solvates having non-pharmaceutically acceptable counterions or associated solvents may be suitable.

The compound of group (II) may be expressed by the following formula:

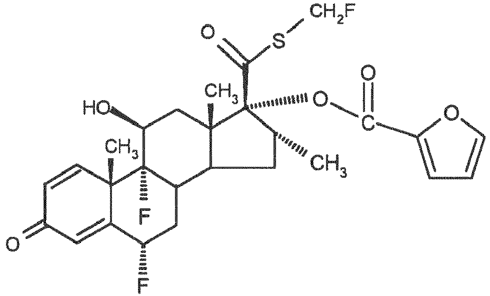

The compound of formula (II) is known as fluticasone furoate ("Compound A")

Compounds of formulas (I) and (II) may be present as part of the same pharmaceutical composition in the inhalation device or may be present in different physical regions within the device, e.g., different reservoirs or strips, as part of separate compositions. As a result, the compounds of formulas (I) and (II) may be administered separately, sequentially, or simultaneously, to a subject in treating a respiratory disorder, using techniques known in the art. Preferably, the compounds are administered simultaneously.

The compositions (e.g., formulations) may be prepared according to methods that are known in the art, as well as components (e.g., inert ingredients) that make up the same. Along with the compounds described herein, such formulations may also include at least one inert ingredient. Inert ingredients are broadly defined to include excipients, carriers, additives that improve stability performance, and the like.

Examples of excipients include mono-saccharides, such as mannitol, arabinose, xylitol and dextrose and monohydrates thereof, disaccharides, such as lactose, maltose and sucrose, and polysaccharides such as starches, dextrins or dextrans. More preferred excipients comprise particulate crystalline sugars such as glucose, fructose, mannitol, sucrose and lactose. Especially preferred excipients are anhydrous lactose and lactose monohydrate.

Generally, the particle size of the excipient particles will be much greater than that of the compound and as a result, do not penetrate into the respiratory tract. Thus, excipient particles for inhalable compositions may typically have particle sizes greater than 20µm, more preferably in the range 20 - 150µm. If desired, the inhalable compositions may also contain two or more excipient particle size ranges. For example, in one embodiment, in order to control the proportion of inhaled medicament, while retaining a good accuracy for metering, it may be desirable to use one component of the excipient that has a particle size of less than 15µm (the fine excipient component) and another component of the excipient that has a particle size of greater than 20µm but lower than 150µm, preferably lower than 80µm (the coarse excipient component).

The proportion of excipient material to be used in the inhalable compositions of this invention (e.g., lactose) may vary depending upon the proportion of each active agent, the powder inhaler for administration etc. The proportion may, for example, be about 75% to 99.5% by weight of the composition as a whole. In other embodiments, the excipient material may range from 94 to 99%, e.g. 97.7 to 99.0% by weight of the composition as a whole. In addition, the dry powder pharmaceutical formulation may also include an additive which improves stability performance, e.g. magnesium stearate or calcium stearate. Where such additives are present, in one embodiment, their concentration may range from 0.1 % w/w to 2.0% w/w. In various embodiments, their concentration may range of 0.2 to 2%, e.g. 0.6 to 2%%, e.g. 0.75%, 1%, or e.g., 1.25% or 1.5% w/w. In one embodiment, the magnesium stearate will typically have a particle size in the range 1 to 50µm, and more particularly 1 - 20µm, e.g.1-10µm.

In one embodiment, a preferred composition consists of the compounds (I) and (II), (i.e., compounds B and A), lactose and magnesium stearate, typically present in the amounts set forth herein.

The dry powder formulations in accordance with the present invention can be prepared according to standard methods. As an example, pharmaceutically active agent or agents and inert ingredient can be intimately mixed using any suitable blending apparatus, such as high shear blenders. The particular components of the formulation can be admixed in any order. Pre-mixing of particular components may be found to be advantageous in certain circumstances. The progress of the blending process can be monitored by carrying out content uniformity determinations. For example, the blending apparatus may be stopped, materials removed using a sample thief and then analyzed for homogeneity by High Performance Liquid Chromatography (HPLC).

The dry powder compositions for use in accordance with the present invention are administered via inhalation devices. As an example, such devices can encompass capsules and cartridges of, for example gelatin, or blisters of for example laminated aluminum foil. In various embodiments, each single or multi-dose capsule, cartridge, or blister may contain the one or more pharmaceutical compositions therein in doses of compounds according to the teachings provided herein. Examples of inhalation devices can include those intended for unit dose or multi-dose delivery. In the case of multi-dose delivery, the formulation can be pre-metered (e.g., as in Diskus®, see GB 2242134/ U.S. Patent Nos. 6,032,666, 5,860,419, 5,873,360, 5,590,645, 6,378,519 and 6,536,427 or Diskhaler, see GB 2178965, 2129691 and 2169265, US Pat. Nos. 4,778,054, 4,811,731, 5,035,237) or metered in use (e.g. as in Turbuhaler, see EP 69715, or in the devices described in U.S. Patent No 6,321,747). An example of a unit-dose device is Rotahaler (see GB 2064336). In one embodiment, the Diskus^{®} inhalation device comprises an elongate strip formed from a base sheet having a plurality of recesses spaced along its length and a lid sheet peelably sealed thereto to define a plurality of containers, each container having therein one or more inhalable formulations containing the compounds optionally with other excipients and additives taught herein. The peelable seal is an engineered seal, and in one embodiment the engineered seal is a hermetic seal. Preferably, the strip is sufficiently flexible to be wound into a roll. The lid sheet and base sheet will preferably have leading end portions which are not sealed to one another and at least one of the leading end portions is constructed to be attached to a winding means. Also, preferably the engineered seal between the base and lid sheets extends over their whole width. The lid sheet may preferably be peeled from the base sheet in a longitudinal direction from a first end of the base sheet.

A dry powder composition may be presented in an inhalation device which permits separate containment of the two different compounds of (I) and (II) in two discrete pharmaceutical compositions. Thus, for example, these compounds may be administrable simultaneously but are stored separately, e.g. in the two separate pharmaceutical compositions, for example as described in WO 03/061743 A1 WO 2007/012871 A1 and/or WO2007/068896. In one embodiment an inhalation device permitting separate containment of components is an inhaler device having two peelable blister strips, each strip containing pre-metered doses in blister pockets arranged along its length. As an example, one blister strip has multiple containers (e.g., blisters) of composition containing the compound of formula (I), and the other blister strip has multiple containers (e.g., blisters) of another composition containing the compound of formula (II). Said device has an internal indexing mechanism which, each time the device is actuated, peels opens a pocket of each strip and positions the blisters so that each newly exposed dose of each strip is adjacent to the manifold which communicates with the mouthpiece of the device. When the patient inhales at the mouthpiece, each dose is simultaneously drawn out of its associated pocket into the manifold and entrained via the mouthpiece into the patient's respiratory tract. A further device that permits separate containment of different components is DUOHALER™ of Innovata. In addition, various structures of inhalation devices provide for the sequential or separate delivery of the pharmaceutical compositions from the devices, in addition to simultaneous delivery.

In another aspect, the invention includes methods of treating respiratory disorders. The method of treating a respiratory disorder includes administering to a patient a therapeutically effective amount of one or more (e.g., two) dry powder pharmaceutical compositions by oral inhalation, comprising a compound of (I) 4-{(1*R*)-2-[(6-{2-[(2,6-dichlorobenzyl)oxy]ethoxy}hexyl)amino]-1-hydroxyethyl}-2-(hydroxymethyl) phenol, or a salt thereof, and a compound of (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof using the drug product defined herein of the first aspect of the invention. The method of treatment can be administered via an inhalation device that contains the dry powder compositions present therein as set forth above, and the compounds of (I) and (II) can be administered separately, sequentially or simultaneously.

The term "treatment" is to be construed as encompassing the amelioration or management of, without limitation, any of the respiratory disorders set forth herein, wherein treatment may be construed to include prophylaxis. Examples of respiratory disorders include diseases associated with reversible airways obstruction such as asthma, chronic obstructive pulmonary diseases (COPD) (e.g. chronic and wheezy bronchitis, emphysema), respiratory tract infection and upper respiratory tract disease (e.g., rhinitis, including seasonal and allergic rhinitis).

The compounds administered according to the method described herein above may be administered once-daily, or multiple times per day (e.g., twice, three-times, etc). In various embodiments, the dose per administration for the compounds (I) and (II) may range from 3µg to 800µg. In one embodiment, the compound of (I) may be administered from about 1µg to 200 µg/day, for example 3, 6.25, 12.5, 25, 50, 100 or 200 µg/day (calculated as the free base). In one embodiment Compound (I) may be administered by inhalation at a dose of 12.5 µg/day. In another embodiment Compound (I) may be administered by inhalation at a dose of 25 µg/day. In another embodiment Compound (I) may be administered by inhalation at a dose of 50 µg/day. In one embodiment, the compound of (II) may be administered by inhalation at a dose of from about 25µg to about 800µg daily, and if necessary in divided doses. Thus, in various embodiments, the daily dose of compound (II) may be for example 25, 50, 100, 200, 300, 400, 600 or 800 µg.

In a preferred embodiment, an inhalation device as taught by WO 03/061743 A1 WO 2007/012871 A1 and/or WO2007/068896 is used containing two strips of 30, 14 or 7 regularly distributed blisters. Examples of three compositions for each of the dual strips in such a device may be as follows:

### Composition I:

First Strip: Compound A: 50mcg (micronized), lactose monohydrate: to 12.5mg
Second Strip: Compound B: 40mcg¹ (micronized), lactose monohydrate: to 12.5mg, magnesium stearate: 125 mcg

### Composition II:

First Strip: Compound A: 100mcg (micronized), lactose monohydrate: to 12.5mg
   1 Equivalent to 25 mcg base
Second Strip: Compound B: 40mcg¹ (micronized), lactose monohydrate: to 12.5mg, magnesium stearate: 125 mcg

### Composition III:

First Strip: Compound A: 200mcg (micronized), lactose monohydrate: to 12.5mg
Second Strip: Compound B: 40mcg¹ (micronized), lactose monohydrate: to 12.5mg, magnesium stearate: 125 mcg

In various embodiments, the drug product of the present invention may include a package for storage of the inhalation device described herein, and the package may be formed from a material capable of controlling the ingress of moisture thereto or egress or moisture therefrom. Such a package for storage of a container is often referred to as an overwrap, although other embodiments are contemplated within the scope of the invention. As an example, a drug product may be free of an overwrap. Examples of such packages are described in WO 01/98174. In one embodiment, the package may be impermeable or substantially impermeable to moisture (i.e., less than 0.1 mg/day). In another embodiment, the package may control the ingress or egress of moisture such that the ambient moisture content within the package is essentially constant, such as varying by no more than ±20% RH, preferably by less than ±10% RH over a 2 year shelf life. Ambient moisture content may for example be measured as the relative humidity within the package. In another embodiment, the package may enable moisture transfer in one way only i.e. ingress only or egress only. In another embodiment, the package may enable moisture transfer to either a set minimum /maximum moisture content within the package or within a set minimum / maximum moisture transfer rate.

In one embodiment, the package may be wrappable and sealable around the inhalation device to form an enclosed volume in which the device is present. Such techniques of wrapping and sealing are known in the art. As an example, the sealing comprises heat sealing said packaging material. In other aspects, the seal is formed by ultrasonic welding, heat stamping, adhesive or laser welding methods.

In a further embodiment, the package may have a preformed tray into which the inhalation device is placed and a subsequent sealing lid which is affixed thus forming an enclosed volume in which the device is present. Such techniques of preforming and sealing are known in the art. As an example, the preformed tray is an aluminum and polypropylene laminate and the lid is also an aluminum and polymer laminate. Embodiments of the tray and lid laminate include, without limitation, those of 110-160 micron aluminum and 30 micron polypropylene, and 60 micron aluminum and 25 g/m² polymer respectively. Preferably, the preformed tray and lid should be of sufficient size to accommodate an inhalation device and desiccant packet, e.g., a packet of 70 x 50 x 5 mm. An example of a commercial preformed tray and lid is one provided by Amcor of Bristol, United Kingdom. Such an embodiment may be free of an overwrap material.

A preferred embodiment of a preformed tray and lid is provided by Constantia of Weinburg, Austria. With respect to the tray, and in one preferred embodiment, materials are as follows (from outside to inside):

| | |
|---|---|
| Lubricant | 0.7 g/m² |
| Lacquer | 2.5 g/m² |
| Aluminum | 0.11-0.16mm |
| Adhesive | 6 g/m² |
| Polypropylene | 0.030 mm |
| Peel force of 15 mm wide sample when sealed to reference lid laminate | 16.5 N |

With respect to the lid, in one or more embodiments, approved materials and tolerances are as follows (from outside to inside):

| | |
|---|---|
| Lacquer | 0.8 g/m² |
| Aluminum | 0.060 mm |
| Polypropylene based co-extrusion coating | 25 g/m² |
| Total thickness including embossing | 135 µm |
| by micrometer or equivalent | |
| | |
| Peel force of 15 mm wide sample when sealed to reference tray laminate | 16.5 N |

In a preferred embodiment, when the lid and tray are sealed, they are capable of protecting the dry powder inhalation device from excessive humidity throughout its shelf-life, e.g., the package may have an MVTR of 0.55mg/24hr/pack at 30°C. When the package is sealed, it is preferred that the lid and tray should be capable of withstanding a partial vacuum of 14,000 ft equivalent. In a preferred embodiment, when sealed, the tray/lid combination should provide an opening peel force that is no greater than 20N across the entire opening to allow for relative ease of patient opening.

With respect a preferred embodiment of the lid/tray laminate, in regards to the tray, an aluminum layer of 110 microns is considered as a baseline. It is preferred that the lid be an aluminum/polypropylene laminate and that an aluminum layer of 60 microns be considered baseline.

In accordance with the invention, the package for storage includes a hygroscopic material. The hygroscopic material may be incorporated into the product in a number of ways. As an example, in one embodiment, the package includes a hygroscopic material within the enclosed volume. In an embodiment, the inhalation device and the hygroscopic material are sealable within the overwrap. In one embodiment, the hygroscopic material may be included as a free-moving or free-flowing packet within the drug product and overwrap. Examples of hygroscopic materials include, without limitation, those selected from the group consisting of silica gel, zeolite, alumina, bauxite, anhydrous calcium sulphate, calcium oxide activated bentonite clay, water-absorbing clay, molecular sieve and any mixtures thereof including partially hydrated forms which can provide relative humidity within the product pack within specified range. In another embodiment, the overwrap or inhaler device may be lined, coated or impregnated with hygroscopic material. Examples of humectants include, without limitation, glycerol, sorbitol, polyethylene glycol, mono- and oligomeric sugars, sodium pyroglutamate, sodium tripolyphosphate, monopotassium phosphate, lactic acid and urea. Examples of desiccants include, without limitation, montmorillonite clay, silica gel, crystalline aluminosilicates, calcium oxide, calcium sulfate, activated alumina, metal salts and phosphorus com pounds.

The overwrap may be present in the form of flexible packaging material. In various embodiments, the flexible packaging material can be any material which is impervious to or substantially impervious to moisture. The overwrap utilizing such material is typically present as a laminate structure. In one embodiment, the flexible packaging material preferably comprises a non-thermoplastic substrate (such as, for example, a metal foil, e.g. aluminum foil, of 9µm thickness) and a heat sealable layer disposed thereon, and an additional protective layer, such as, for example, a polymer film of polyester. To further reduce moisture ingress, thicker metal films, such as 12 µm to 20 µm, may be used. In various embodiments, the heat sealable layer is usually disposed on the inner surface of the assembled package. In various embodiments, the additional protective layer is usually disposed on the surface opposite the heat sealable layer. In various embodiments, the RH may be measured within the overwrap.

The substrate is preferably formed from aluminum foil. However, other metals for the substrate include, but are not limited to, tin, silver, iron, zinc, or magnesium formed on a sheet by vacuum deposition or sputtering and a carboxyl group-containing polyolefin layer formed on the metal layer by lamination.

The heat sealable layer can be formed from any thermoplastic or thermosetting material such as an ionomer resin, polyolefin, or cycloolefin copolymer. Ionomer resins typically include ionically cross- linked ethylene-methacrylic acid and ethylene acrylic acid copolymers. Properties which distinguish these ionomers resins from other polyolefin heat-sealed polymers are high clarity, high impact resistance, low haze in lamination, tear resistance, abrasion resistance, solid state toughness, and moisture imperviousness. In an embodiment, the heat sealable layer is made out of SURLYN™ (an ionomer resin) or a form of polyethylene to provide sufficient heat sealing properties.

The outer protective layer, if present, can be formed of any material as long as the final laminate has the requisite properties. In one embodiment, as an example, the protective layer (e.g., polyester) is adhesively laminated to the substrate (e.g., aluminum) and the substrate layer in turn is adhesively laminated to the heat sealable layer (e.g., the ionomer film or SURLYN™ (an ionomer resin).

Examples of thicknesses of the three layers include a protective layer of 20 µm to 30 µm; and a substrate layer of 9 µm to 20 µm. For the heat sealable layer, examples of thicknesses range from 40 to 70 µm.

Adhesives may be used to join the respective layers of materials together. The adhesive layers are typically substantially smaller in thickness relative to the thickness of the substrate, heat sealable and/or protective layers which they bond.

Specific embodiments of overwrap materials are as follows:
1) polyethylene terephthalate ("PET") 12µm/Extr/aluminum 9µm/Extr/low density polyethylene ("LDPE") 35µm
2) PET 12µm (17gsm)/adhesive 4 gsm/aluminum 12µm (32 gsm)/adhesive 3 gsm/LDPE without additives 50 µm (46 gsm)

The hygroscopic materials utilize moisture absorbing materials, such as desiccants and humectants, and a moisture absorbing material is preferably present in the form of a silica gel desiccant packet. However, other vapor or moisture absorbing mechanisms are not beyond the scope of the present invention. Other vapor or moisture absorbing materials include desiccants and humectants made from inorganic materials such a zeolites and aluminas. Such inorganic vapor or moisture absorbing materials have high water absorption capacities and favorable water absorption isotherm shapes. The water absorption capacity of such materials typically varies from 20 to 50 weight percent and the percentage moisture content which controls the relative humidity inside the product pack within a specified range typically varies from 3 to 20 weight percent. Silica gel is particularly suited for enabling hydration between, at a lower end, any of the values,15, 16, 17, 18 19, 20, 21, 22, 23, 24, 25, 26, 27, 28, 29, 30, 31, 32, 33, 34, 35, 36, 37, 38, 39 or 40 to, at an upper end, any of the values, 17, 18, 19, 20, 21, 22, 23, 24, 25, 26, 27, 28, 29, 30, 31, 32, 33, 34, 35, 36, 37, 38, 39, or 40% RH as silica gel is capable of adsorbing up to 35% of its own weight in moisture (preferably up to 30%) and thus is capable of maintaining %RH levels up to 50%. Preferred silica gel packets can be present as Eq-Pak^{®} fan folded packets or Eq-Pak^{®} individually cut packets made commercially available by Sud-Chemie of France. In a preferred embodiment, the absorbing material is present inside TYVEK^{®}, which is a nylon mesh bonded with HDPE fibers, and made commercially available by E.I. Dupont de Nemours of Wilmington, Delaware.

In various embodiments, the term "partial hydration" refers to a hygroscopic material being less than fully saturated. For the purposes of the invention, in one embodiment, the hygroscopic material (e.g., silica gel) may have a moisture content range of from, at a lower end, any of the values, 8.4, 8.8, 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w to, at a higher end, any of the values 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w. Such range includes all singular values mentioned above, i.e., the moisture content may be each of 8.4, 8.8, 9.2% , etc, through 18.6%. The above values are expressed as %w/w of total mass based on the material being completely dry at 0%w/w and fully saturated at around 30%-31% (wt. water/wt. hygroscopic material), in another embodiment, such range can be expressed as the Percentage Relative Humidity range of controlled pack, usually less than 500cc in volume, from at a lower end, any of the values, 15, 16, 17, 18 19, 20, 21, 22, 23, 24, 25, 26, 27, 28, 29, 30, 31, 32, 33, 34, 35, 36, 37, 38, 39 or 40 to, at an upper end, any of the values, 17, 18, 19, 20, 21, 22, 23, 24, 25, 26, 27, 28, 29, 30, 31, 32, 33, 34, 35, 36, 37, 38, 39, or 40 %RH, inclusive of the entire range, including all singular values and ranges set forth herein.

Preferred embodiments of desiccant packets are 50x70x5mm TYVEK^{®} bags containing 2-10 grams (e.g., 4 or 8 grams) of partially hydrated silica gel (Eq-pak^{®}). The Eq-pak^{®} packets may provide between 10 to 40%RH within the product pack. These packets may come in two forms: (1) fan-folded, which is of strip of connected packets of approximately 1000 per bag and (2) individually-cut, which are supplied in bags of 100 each.

Specific embodiments of desiccants **A, B** and **C** are set forth in Tables 1-3:

**Table 1**

| | |
|---|---|
| **"A"** | Eq-Pak 30%RH, |
| **Absorber Material** | Silica Gel |
| **Size/Capacity** | Net Weight: 8.0g (+/- 10%) of Silica Gel Relative Humidity in Packaging: 30%RH (+/-3%) @ 20∘C(+/-3∘C) |
| **Misc** | Residual Moisture Level: 15.8% w/w (+/-1%) |
| **Container Material (Desiccant Packet)** | Tyvek® |
| **Dimensions** | Length: 70mm |
| | Width: 50mm |

**Table 2**

| | |
|---|---|
| **"B"** | Eq-Pak 20%RH |
| **Absorber Material** | Silica Gel |
| **Size/Capacity** | Net Dry Weight: 8.0g (+/- 10%) of Silica Gel Relative Humidity in Packaging: 20%RH (+/-3%) @ 20∘C(+/- 3∘C) |
| **Misc** | Residual Moisture Level: 11.3% w/w (+/-1%) |
| **Container Material (Desiccant Packet)** | Tyvek® |
| **Dimensions** | Length: 70mm |
| | Width: 50mm |

**Table 3**

| | |
|---|---|
| **"C"** | Eq-pak 10%RH |
| **Absorber Material** | Silica Gel |
| **Size/Capacity** | Net Dry Weight: 8.0g (+/- 10%) of Silica Gel Relative Humidity in Packging: 10%RH (+/-3%) @ 20∘C(+/-3∘C) |
| **Misc** | Residual Moisture Level: 6.8% w/w (+/-1%) |
| **Container Material (Desiccant Packet)** | Tyvek® |
| **Dimensions** | Length: 70mm |
| | Width: 50mm |

The RH of desiccant packets may be measured by sealing a desiccant packets inside a small foil laminate pouch then inserting a pointed, narrow RH probe into the pouch and recording the RH when a stable value is attained (i.e., little or no variation), which as an example, typically occurs in two minutes. Typically a Rotronic Hygropalm meter with SC04 probe is used, although other measuring devices/techniques can be used.

Other exemplary moisture absorbing materials include, but are not limited to, alumina, bauxite, anhydrous, calcium sulphate, water-absorbing clay, activated bentonite clay, a zeolite molecular sieve, or other like materials which optionally include a moisture sensitive colour indicator such as cobalt chloride to indicate when the desiccant is no longer operable. In various embodiments, the RH may be measured within the overwrap.

The desiccant should be present in an amount sufficient to absorb any excess moisture inside the package. When silica gel is used, and in one example 1 g to 12g of silica gel is sufficient for a typical dry powder inhaler. Moreover, the desiccant can be present in an amount sufficient to absorb any moisture that possibly ingresses from the external environment or that is present in the polymers used to fabricate the device. It is also possible to place the desiccant adjacent to the inhalation device. As an example, in embodiments in which an overwrap material envelops the inhalation device, the desiccant may be present therein in a loose or free-flowing manner. In other embodiments, the desiccant can be secured to the inside of the overwrap by structures know in the art, such as those disclosed in WO 01/98174.

In various embodiments, the term "partial hydration" refers to a hygroscopic material being less than fully saturated. For the purposes of the invention, in one embodiment, the hygroscopic material (e.g., silica gel) may have a moisture content range of from at a lower end, any of the values, 8.4, 8.8, 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w to, at a higher end, any of the values, 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w of total mass based on the material being completely dry at 0%w/w and fully saturated at around 30%-31% (wt. water/wt. hygroscopic material), in another embodiment, such range can be expressed as the Percentage Relative Humidity range of controlled pack, usually less than 500cc in volume, from, at a lower end, any of the values, 15, 16, 17, 18 19, 20, 21, 22, 23, 24, 25, 26, 27, 28, 29, 30, 31, 32, 33, 34, 35, 36, 37, 38, 39 or 40 to, at an upper end, any of the values, 17, 18, 19, 20, 21, 22, 23, 24, 25, 26, 27, 28, 29, 30, 31, 32, 33, 34, 35, 36, 37, 38, 39, or 40% RH. Such ranges include all other singular values and ranges set forth herein.

In another aspect, the invention provides a process of producing a drug product. The process comprises subjecting a hygroscopic material to moisture exposure (e.g., water or humidity) sufficient to attain a predetermined moisture content and thereby a predetermined relative humidity (e.g., via partial hydration as described herein); combining the hygroscopic material with an inhalation device including one or more pharmaceutical compositions containing (I) 4-{(1*R*)-2-[(6-{2-[(2,6-dichlorobenzyl)oxy]ethoxy}hexyl)amino]-1-hydroxyethyl}-2-(hydroxymethyl) phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; wherein each of the active ingredients (I) and (II) are present in the same or different compositions and then enclosing the dry powder inhalation device and hygroscopic material within the package to define an enclosed volume therein forming a drug product. The enclosed volume may have an %RH of from at a lower end, any of the values, 15, 16, 17, 18 19, 20, 21, 22, 23, 24, 25, 26, 27, 28, 29, 30, 31, 32, 33, 34, 35, 36, 37, 38, 39 or 40%RH to, at an upper end, any of the values, 17, 18, 19, 20, 21, 22, 23, 24, 25, 26, 27, 28, 29, 30, 31, 32, 33, 34, 35, 36, 37, 38, 39, or 40%RH, inclusive of all singular values and ranges taught herein. Such moisture exposure conditions relating to predetermined relative humidity exposure are described herein. The invention also includes drug products made by such processes. The RH within the moisture protective container enclosing the drug product may be measured by inserting a pointed, narrow RH probe into the container and recording the RH when a stable value is attained. Typically a Rotronic Hygropalm meter with SC04 probe is used, although other devices/techniques can be used.

To further elaborate on the above, the dry powder inhalation device and hygroscopic material are combined by being brought together in sufficient proximity such that they may be packaged using accepted techniques, e.g., in a preferred embodiment the device and hygroscopic material are placed in a tray and thereafter are enclosed by placement of a lid over the tray opening. The present invention is highly advantageous. By virtue of the elements of the drug product, the dry powder composition may be capable of exhibiting a fine particle mass (FPM) that is relatively stable, e.g., in one embodiment may deviate no more than +/- 15% from 30% of nominal total drug content per blister (ie. 25.5 to 34.5%) for compound (I) and no more than +/- 15% from 20% of nominal total drug content per blister (i.e. 17-23%) for compound (II) over the product shelf-life of 2 years when the product is stored at 50 to 60% RH and 20°C to 25°C, and in another embodiment, when the product is stored at 75% RH and 30°C.

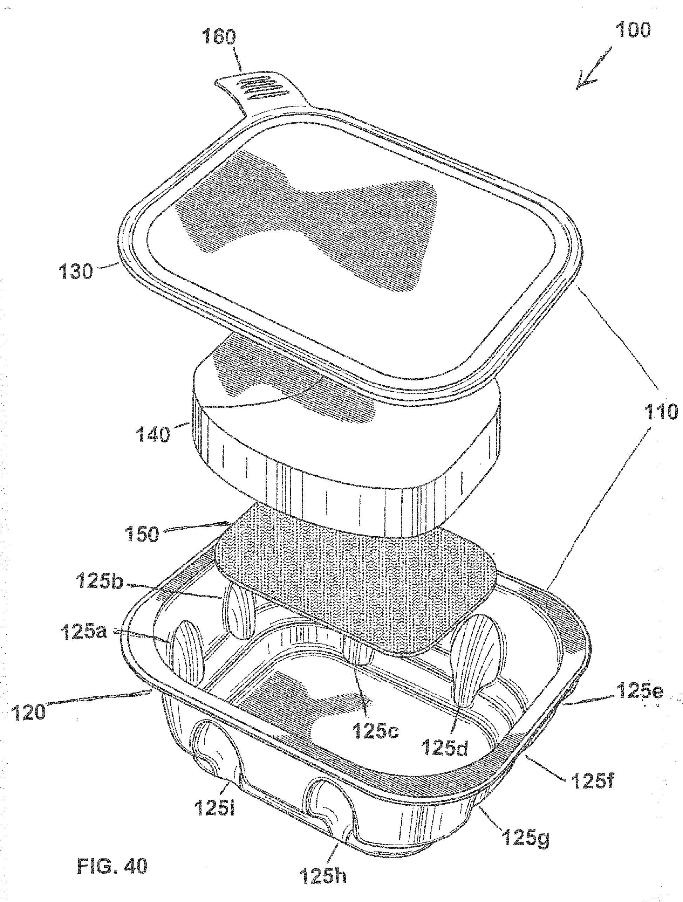

**FIG. 40** is a drawing representing a preferred embodiment of a drug product **100** according to the present invention. It should be appreciated that other embodiments are encompassed by the scope of the present invention and that this specific embodiment does not serve to limit that scope.

As shown, drug product **100** includes a package **110** in the form of a tray **120** and lid **130.** The tray **120** is conformed to receive a dry powder inhalation device **140** therein. Ribs **125a, 125b, 125c, 125d, 125e, 125f, 125g, 125h** and **125i** are present to minimize or prevent movement of device **140** therein. Also illustrated between device **140** and tray **120** is hygroscopic material **150.** In this embodiment, hygroscopic material **150** is present in the form of a loose desiccant packet. Lid **130** serves to enclose device **140** and hygroscopic material **150** within the tray **120** when sealed to the tray. As depicted, lid **130** contains a tab **160** to allow for relative ease of removal of the lid **130** from the tray **120.** Device **140** may thereafter be used in a conventional fashion.

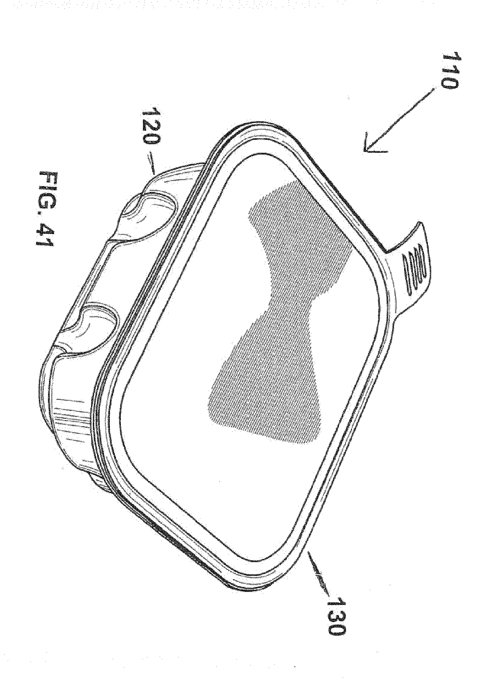

**FIG. 41** shows the lid **130** sealed to the tray **120** with device and hygroscopic material contained therein.

The invention will now be described with respect to the following examples. It should be appreciated that these examples are set forth for the purpose of illustrating the invention, and does not limit the scope of the invention as defined by the claims.

### Definition of terms

The following terms are used herein:
"Inhalation grade lactose" is comprised of alpha lactose monohydrate having a predetermined shape and particle size distribution. A source of inhalation grade lactose is Friesland Campina Domo in the Netherlands.
"Foil laminate 1" as used herein consists of a layered sheet of oriented polyester teraphthalate (12µm), aluminum foil (9µm), and low density polyethylene (35µm).
"Foil laminate 2" as used herein consists of a layered sheet of oriented polyester teraphthalate (12µm), adhesive (4gsm), aluminum foil (12µm), adhesive (3gsm), and low density polyethylene (50µm).
"Standard blending equipment" indicates that blending was performed using high shear blenders at a scale of 4-35kg. Examples of high shear blender are PMA and TRV high speed blender.
"Standard filling equipment" indicates that filling was performed using equipment described in U.S. Patent No. 5,187,921A or U.S. Publication No. 2005-0183395.
"Device" as used herein in the examples refers to inhalation device described in U.S. Publication No. 2008-0308102 A1.
"Standard stability chamber" are environmental chambers that can attain temperature accuracy and precision of +/- 2°C and a RH accuracy and precision of +/- 5%RH.
"Cascade impaction" refers to Apparatus 5 in *General Chapter <601> Aerosols, Nasal Sprays, MDIs* & *DPIs: Uniformity of Dosage Units* (MDI & DPI) USP30 used at 60l/min for a duration of 4 seconds per actuation. Typically 10-20 actuations are discharged per determination. "Fine Particle Mass" is defined as the sum of the deposition on stage 2 to stage 5 and is calculated as a percentage of the nominal drug target contained in each individual blister.
"HPLC" refers to standard high performance liquid chromatography.
"UV/visible detection" indicates an ultraviolet detector at the outlet of the HPLC.
"Fluorescence detection" indicates a fluorescence detector at the outlet of the HPLC.
"Lactose fines" are the proportion of the particle size distribution less than 15µm, measured by laser diffraction of a suspension (dry dispersion), or less than 4.5µm of an air dispersion (dry dispersion).
Compound "A" refers to (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate.
Compound "B" refers to 4-{(1*R*)-2-[(6-{2-[(2,6-dichlorobenzyl)oxy]ethoxy }hexyl)amino]-1-hydroxyethyl}-2-(hydroxymethyl)phenol triphenyl acetate. The quantities of Compound B quoted in the examples is for the free base.
Unprotected product refers to a product unprotected from moisture and has been used interchangeable with the term 'naked'.

### Example 1

### Relationship of desiccant, humidity and temperature to inhalable formulation fine particle mass

The comparison of dual active formulated product, stored in foil laminate 1 (overwrapped) or in foil laminate 1 with a desiccant (overwrapped with desiccant) is illustrated herein as it relates to total fine particle mass as a function of storage time, relative humidity and temperature.

Blends were manufactured and filled using standard equipment. Blends were packaged in strips as individual actives and in devices as dual active products (i.e., one strip of each active). The blends consisted of four levels of active ingredient, 50µg and 800µg of Compound A and the balance to 12.5mg inhalation grade lactose (lactose fines 7 % and 4% < than 4.5 microns by dry dispersion respectively), and 100µg and 6.25µg of Compound B, 1% magnesium stearate, and the balance to 12.5mg of inhalation grade lactose (lactose fines 8% <15 microns by wet dispersion). Final packaged device drug contents were 6.25µg/50µg, 6.25µg /800µg, 100µg/50µg and 100µg /800µg of Compound B and Compound A, respectively (henceforth referred to as 6.25/50, 6.25/800, 100/50 and 100/800). Devices were overwrapped or overwrapped with a desiccant packet. Desiccant consisted of an 8g 20% RH Eq-pak^{®}. The relative humidity within the overwrapped packs without desiccant was around 45%RH. Overwrapped devices were subsequently stored in standard stability chambers and storage conditions were 25°C and 60% RH, 30°C and 75% RH and 40°C and 75% RH. Samples were analyzed for fine particle mass at time intervals up to 24 months for samples stored at 25°C and 60% RH and up to 6 months for samples stored at 30°C and 75% RH and 40°C and 75% RH. Fine particle mass was determined using cascade impaction with HPLC coupled to UV/visible and/or fluorescence detection.

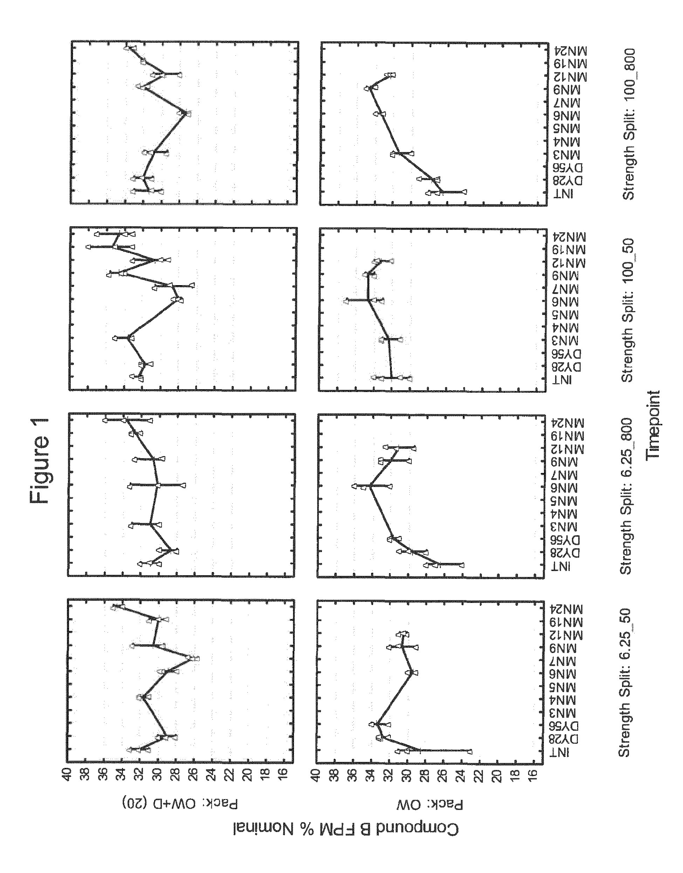

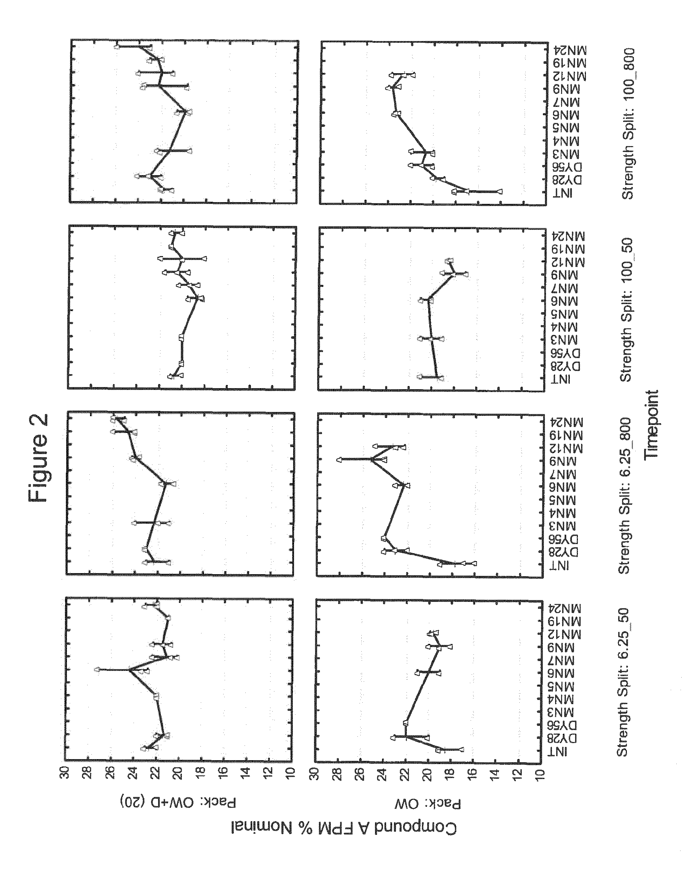

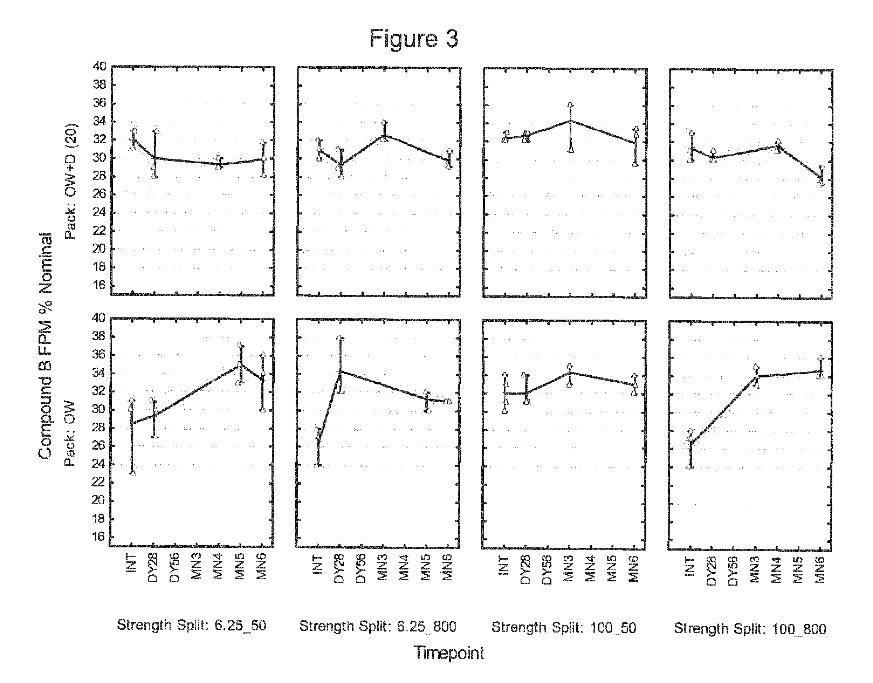

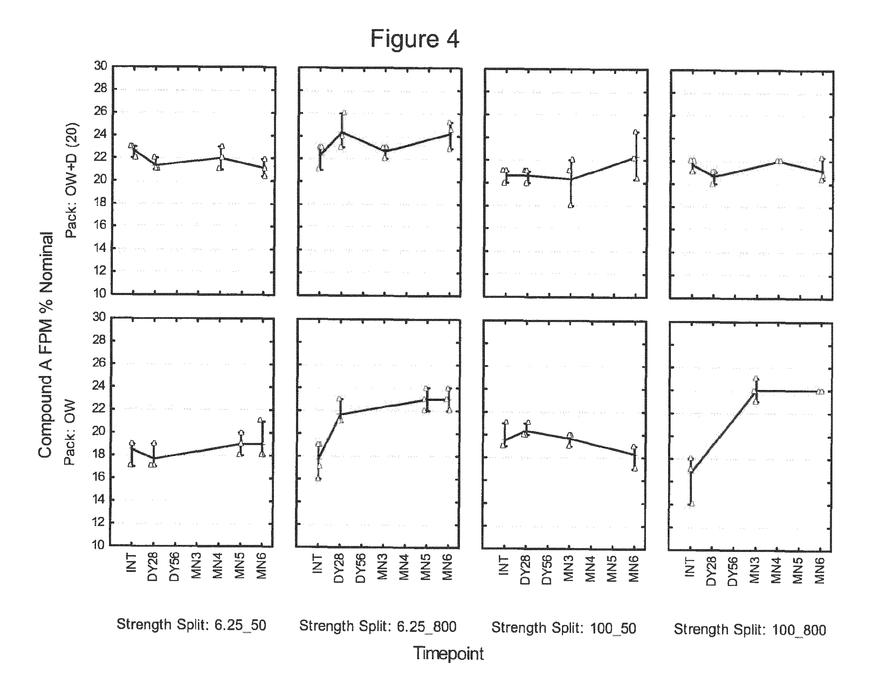

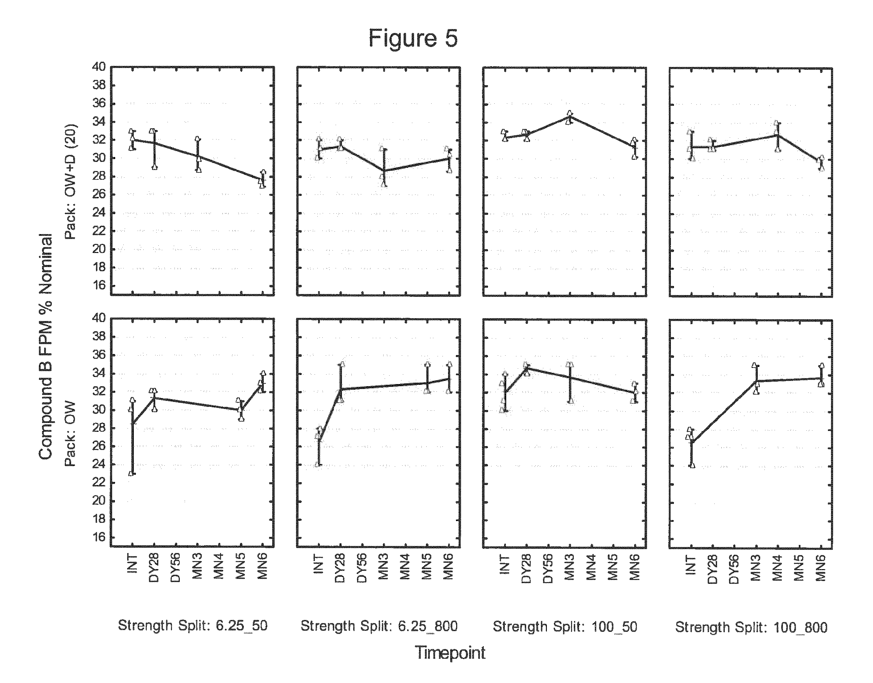

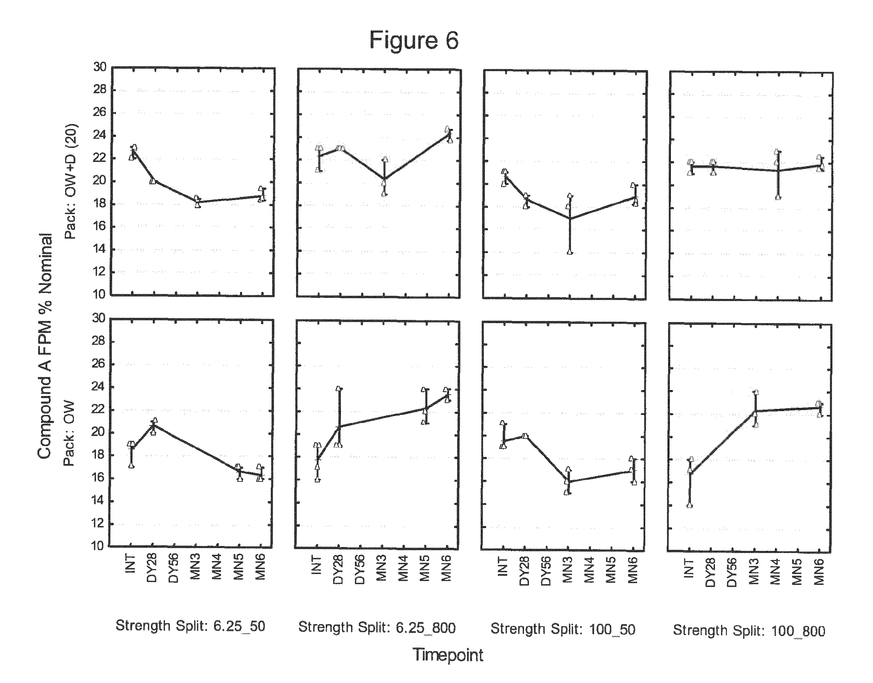

Figures 1-6 are illustrative of the changes in total fine particle mass as a percentage of the nominal drug per dose (%nominal) as a function of both desiccant and storage conditions at select time intervals for the 6.25/50, 6.25/800, 100/50 and 100/800 samples overwrapped (OW) and overwrapped with desiccant (OW+D). Figures 1, 3 and 5 show the changes in fine particle mass (% Nominal) for blends 6.25/50, 6.25/800, 100/50 and 100/800 of Compound B at 25°C/60% RH, 30°C/75% RH and 40°C/75% RH, respectively while Figures 2, 4 and 6 show the changes in fine particle mass (% Nominal) for blends 6.25/50, 100/50, 6.25/800 and 100/800 for Compound A at 25°C/60% RH, 30°C/75% RH and 40°C/75% RH respectively.

### Example 2

### Relationship of desiccant, humidity and temperature to inhalable formulation fine particle mass

The comparison of product stored unprotected (naked), packed in foil laminate 2 (overwrapped) and packed in foil laminate 2 with desiccant (overwrapped with desiccant) providing a range of relative humidities (RH) within the pack is illustrated herein as it relates to fine particle mass as a function of storage conditions over time.

Blends were manufactured and filled using standard equipment. The inhalation device contained two packs in peelable blister strip form, the first containing blend with 12.5 µg of compound B, 1% magnesium stearate, and the balance to 12.5mg of inhalation grade lactose (lactose fines 4.5% < than 4.5 microns by dry dispersion) and the second peelable blister strip containing 50 µg of compound A and the balance to 12.5mg of inhalation grade (lactose fines 6.5% < than 4.5 microns by dry dispersion) lactose. Samples were left unprotected, overwrapped or overwrapped with a desiccant packet. Desiccant humidity values were 15% (8g Eq-pak^{®}, 10% RH plus 8g Eq-pak^{®}, 20% RH), 20% (8g Eq-pak^{®}, 20% RH)and 30% (8g Eq-pak^{®}, 30% RH). The RH inside the OW packs was around 45% RH. Overwrapped samples and samples overwrapped with desiccant were subsequently stored in standard stability chambers and storage conditions were 25°C and 60% RH and 40°C and 75% RH. Unprotected samples were stored in standard stability chambers and storage conditions were 25°C and 75% RH. Samples were analyzed for fine particle mass initially and at time intervals up to 3 months for unprotected product stored at 25°C and 75%RH, up to 6 months for overwrapped and overwrapped with desiccant samples stored at 40°C and 75% RH and up to 9 or 12 months for overwrapped and overwrapped with desiccant samples stored at 25°C and 60% RH. Fine particle mass was determined using cascade impaction with HPLC coupled to UV/visible and fluorescence detection.

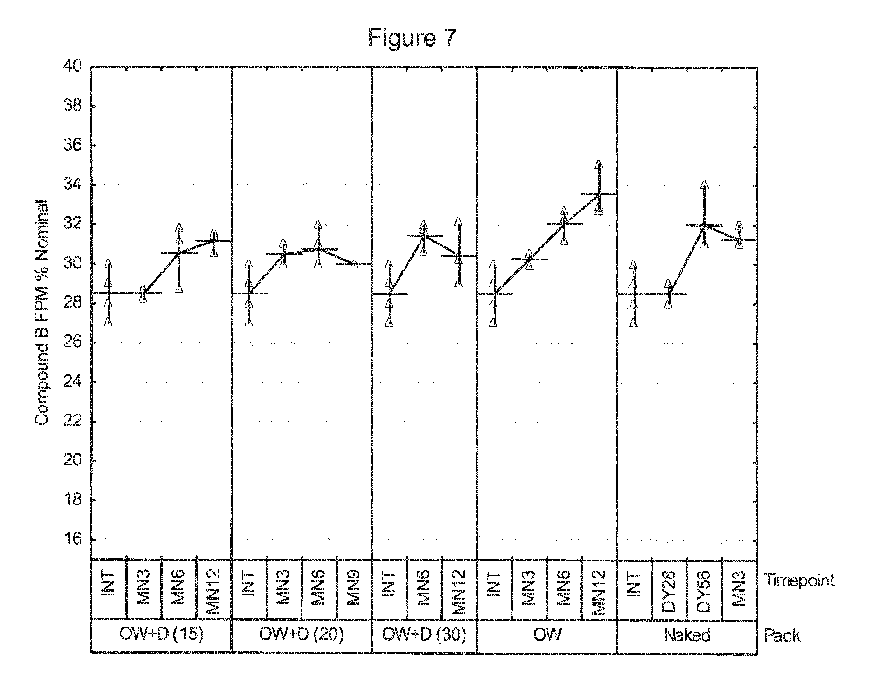

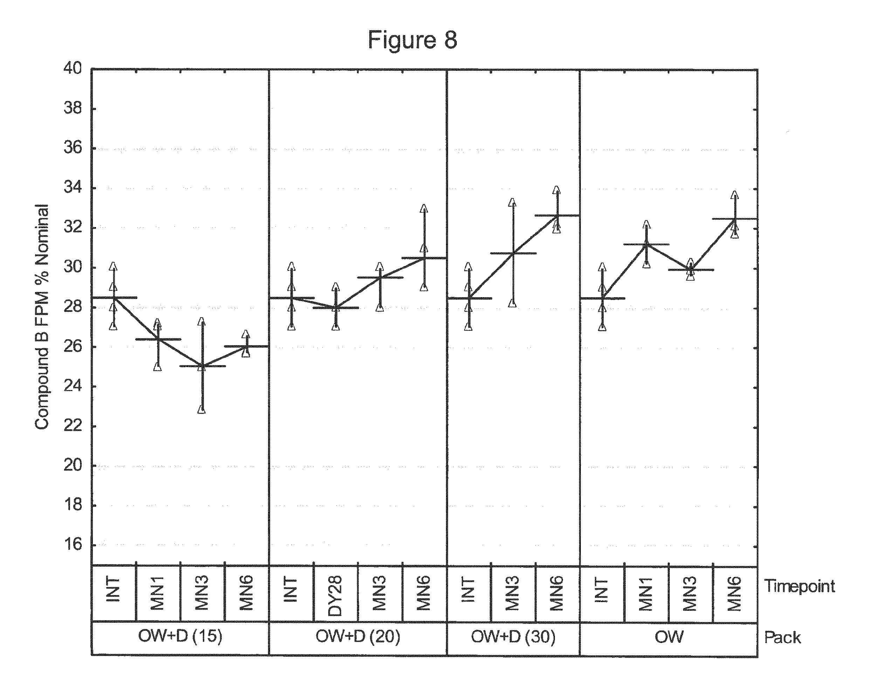

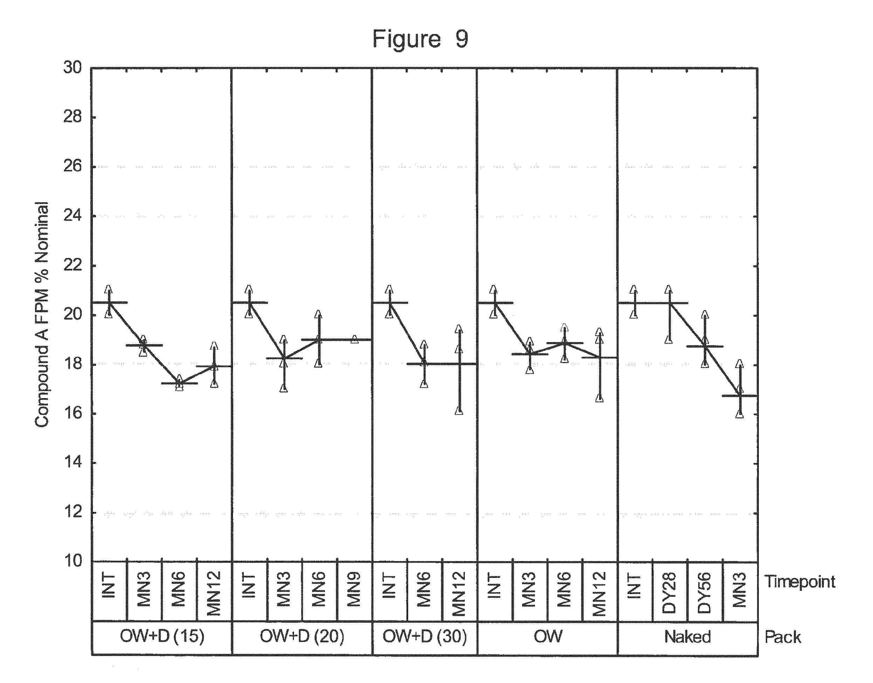

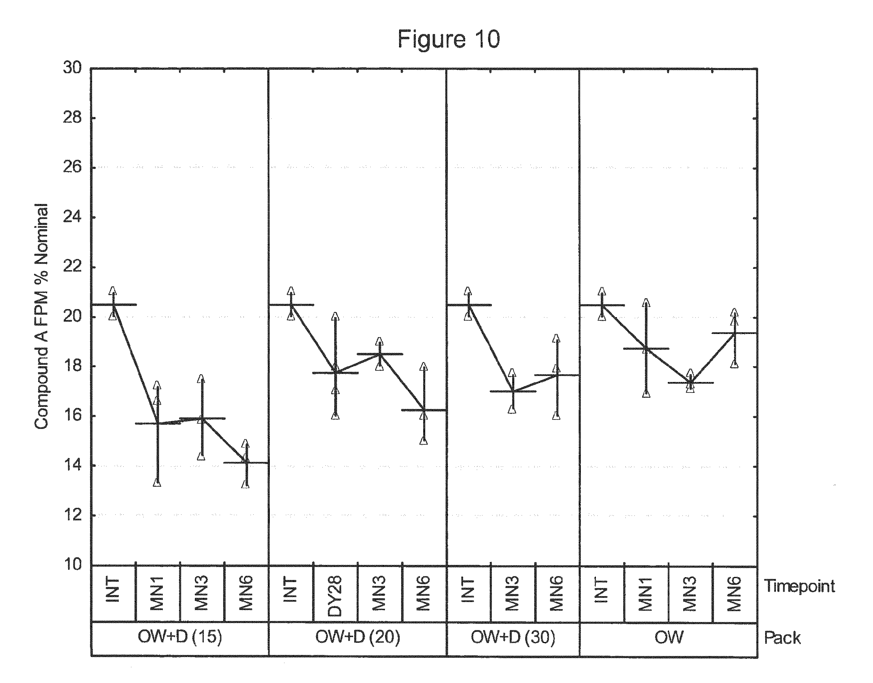

Figures 7-10 are illustrative of the changes in total fine particle mass as a percentage of the nominal drug per dose (%nominal) as a function of both desiccant RH values and storage time. Figures 7 and 9 show fine particle mass values for samples stored overwrapped (OW) and overwrapped with desiccant (OW+D) at 25°C and unprotected at 25°C and 75%RH at select time intervals for compound B and compound A respectively. Figures 8 and 10 show fine particle mass values for samples stored overwrapped (OW) and overwrapped with desiccant (OW+D) at 40°C at select time intervals for compound B and compound A respectively.

### Example 3

### Relationship of desiccant, humidity and temperature to inhalable formulation fine particle mass

The comparison of dual active formulated product, stored in foil laminate 1 with a desiccant (overwrapped with desiccant) or unprotected is illustrated herein as it relates to total fine particle mass as a function of storage time, relative humidity and temperature.

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7 %, 6.2% and 5.7% < than 4.5 microns by dry dispersion), 25µg of Compound B, 1% magnesium stearate, and the balance to 12.5mg of inhalation grade lactose (lactose fines 4.5% < than 4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet or left unprotected. Desiccant consisted of an 10% (8g Eq-pak^{®}, 10% RH), 15% (8g Eq-pak^{®}, 10% RH plus 8g Eq-pak^{®}, 20% RH), 20% (8g Eq-pak^{®}, 20% RH), 30% (8g Eq-pak^{®}, 30% RH) and 40% (8g Eq-pak^{®}, 40% RH). Overwrapped devices were subsequently stored in standard stability chambers and storage conditions were 25°C and 60% RH and 40°C and 75% RH. Samples were analyzed for fine particle mass at time intervals up to 6 months for samples stored at 25°C and 60% RH and up to 7 months for samples stored at 40°C and 75% RH. Fine particle mass was determined using cascade impaction with HPLC coupled to UV/visible and/or fluorescence detection.

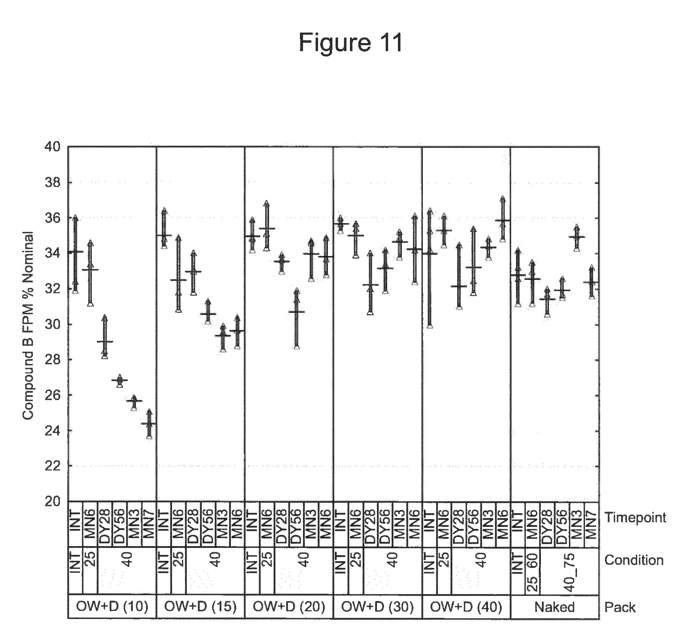

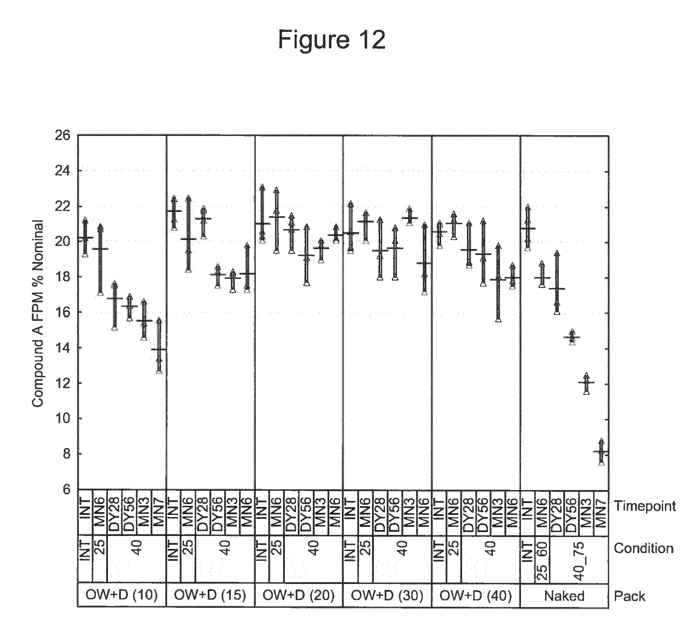

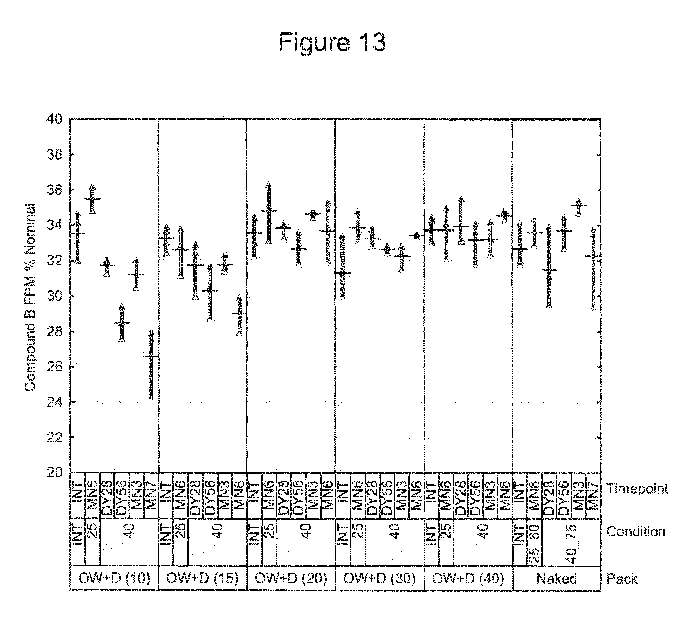

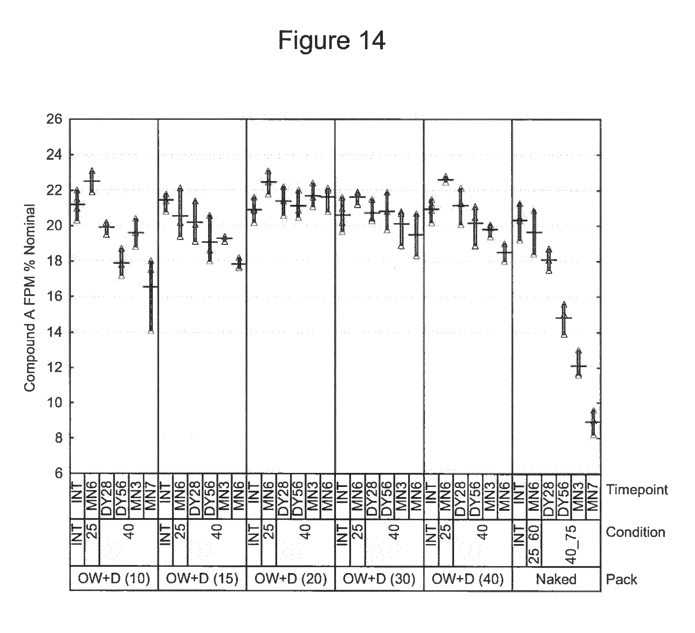

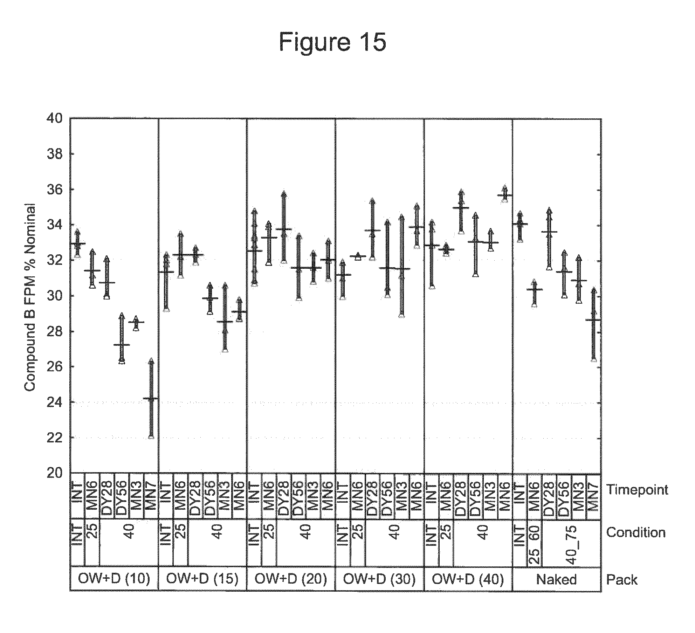

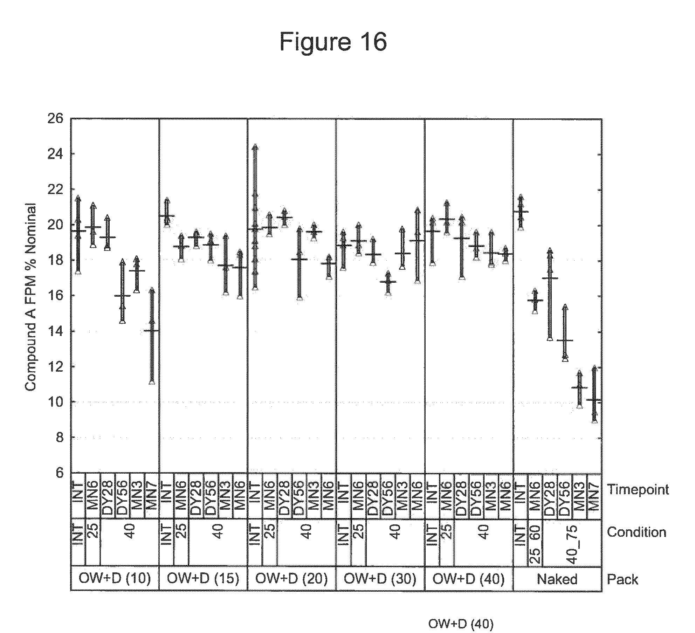

Figures 11-16 are illustrative of the changes in total fine particle mass as a percentage of the nominal drug per dose (%nominal) as a function of both desiccant and storage conditions at select time intervals for the 50/25, 100/25, and 200/25 samples overwrapped with desiccant (OW+D) and unprotected. Figures 11, 13 and 15 show the changes in fine particle mass (% Nominal) for blends 50/25, 100/25 and 200/25 for Compound B at 25°C/60% RH and 40°C/75% RH, respectively while Figures 12, 14 and 16 show the changes in fine particle mass (% Nominal) for blends 50/25, 100/25 and 200/25 for Compound A at 25°C/60% and 40°C/75% RH respectively.

### Example 4

### Relationship of desiccant, humidity and temperature to inhalable formulation mass median aerodynamic diameter (MMAD)

The comparison of product stored unprotected (naked), packed in foil laminate 2 (overwrapped) and packed in foil laminate 2 with desiccant (overwrapped with desiccant) providing a range of relative humidities (RH) within the pack is illustrated herein as it relates to mass median aerodynamic diameter (MMAD) as a function of storage conditions over time.

Blends were manufactured and filled using standard equipment. The inhalation device contained two packs in peelable blister strip form, the first containing blend with 12.5 µg of compound B, 1% magnesium stearate, and the balance to 12.5mg of inhalation grade lactose (lactose fines 4.5% < than 4.5 microns by dry dispersion) and the second peelable blister strip containing 50 µg of compound A and the balance to 12.5mg of inhalation grade (lactose fines 6.5% < than 4.5 microns by dry dispersion) lactose. Samples were left unprotected, overwrapped or overwrapped with a desiccant packet. Desiccant humidity values were 15% (8g Eq-pak^{®}, 10% RH plus 8g Eq-pak^{®}, 20% RH), 20% (8g Eq-pak^{®}, 20% RH) and 30% (8g Eq-pak^{®}, 30% RH). The RH inside the OW packs was around 45% RH. Overwrapped samples and samples overwrapped with desiccant were subsequently stored in standard stability chambers and storage conditions were 25°C and 60% RH and 40°C and 75% RH. Unprotected samples were stored in standard stability chambers and storage conditions were 25°C and 75% RH. Samples were analyzed for MMAD initially and at time intervals up to 3 months for unprotected product stored at 25°C and 75%RH, up to 6 months for overwrapped and overwrapped with desiccant samples stored at 40°C and 75% RH and up to 9 or 12 months for overwrapped and overwrapped with desiccant samples stored at 25°C and 60% RH. MMAD was determined using cascade impaction with HPLC coupled to UV/visible and fluorescence detection.

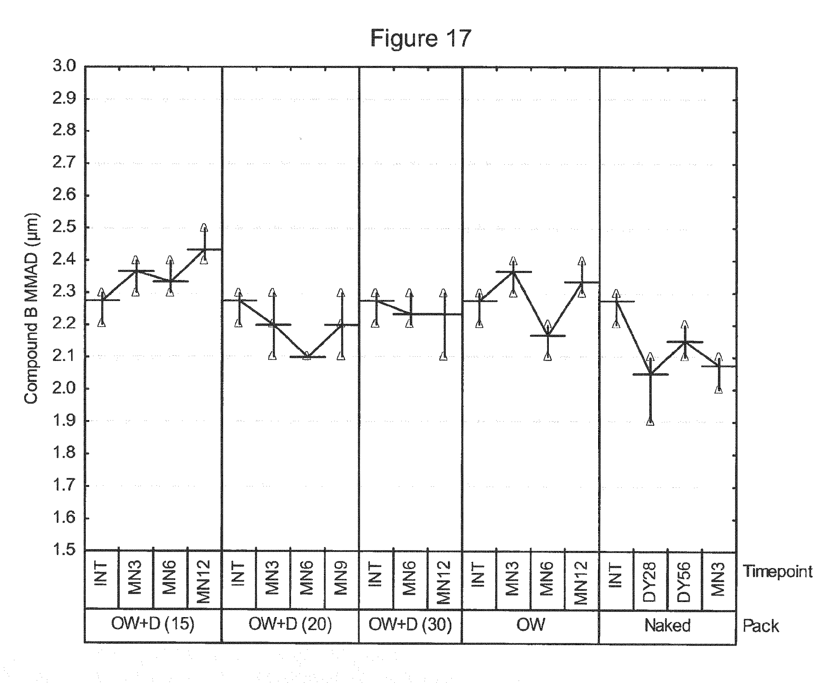

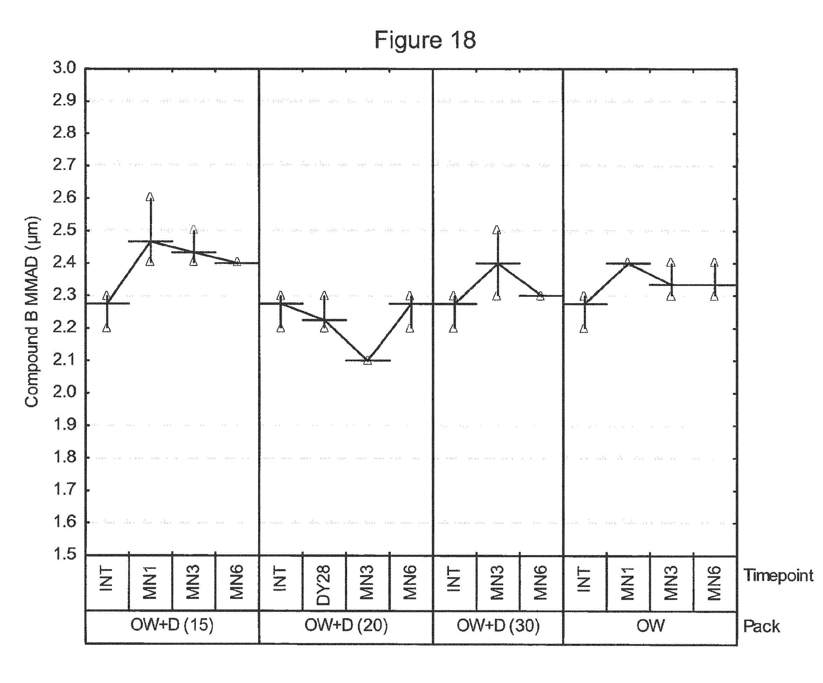

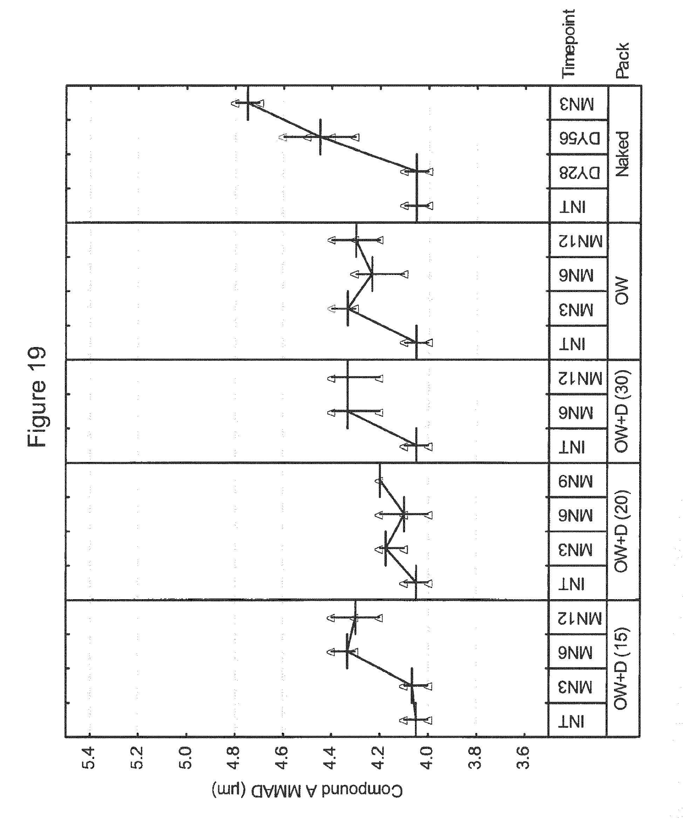

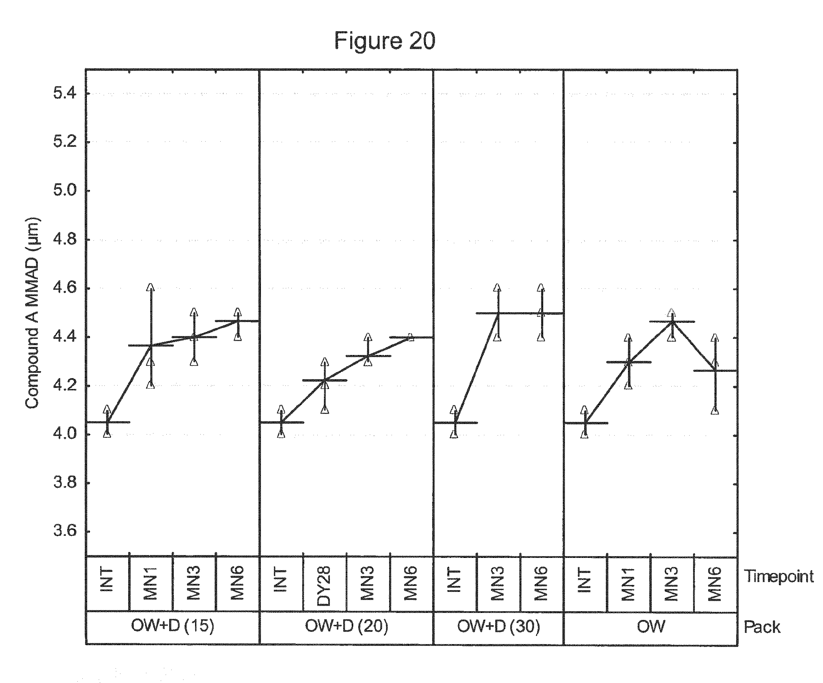

Figures 17-20 are illustrative of the changes in MMAD, in microns, as a function of both desiccant RH values and storage time. Figures 17 and 19 show MMAD values for samples stored overwrapped (OW) and overwrapped with desiccant (OW+D) at 25°C and unprotected (naked) at 25 °C and 75%RH at select time intervals for compound B and compound A respectively. Figures 18 and 20 show MMAD values for samples stored overwrapped (OW) and overwrapped with desiccant (OW+D) at 40°C at select time intervals for compound B and compound A respectively.

### Example 5

### Relationship of desiccant, humidity and temperature to inhalable formulation mass median aerodynamic diameter (MMAD)

The comparison of dual active formulated product, stored in foil laminate 1 with a desiccant (overwrapped with desiccant) or unprotected is illustrated herein as it relates to mass median aerodynamic diameter (MMAD) as a function of storage time, relative humidity and temperature.

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7%, 6.2% and 5.7% < than 4.5 microns by dry dispersion respectively), 25µg of Compound B, 1% magnesium stearate, and the balance to 12.5mg of inhalation grade lactose (lactose fines 4.5% <4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet or left unprotected (naked). Desiccant humidity values were 10% (8g Eq-pak^{®}, 10% RH), 15% (8g Eq-pak^{®}, 10% RH plus 8g Eq-pak^{®}, 20% RH), 20% (8g Eq-pak^{®}, 20% RH), 30% (8g Eq-pak^{®}, 30% RH) and 40% (8g Eq-pak^{®}, 40% RH). Overwrapped and desiccated devices were subsequently stored in standard stability chambers and storage conditions were 25°C and 60% RH and 40°C and 75% RH. Samples were analyzed for mass median aerodynamic diameter (MMAD) at time intervals up to 6 months for samples stored at 25°C and 60% RH and up to 7 months for samples stored at 40°C and 75% RH. MMAD was determined using cascade impaction with HPLC coupled to UV/visible and fluorescence detection.

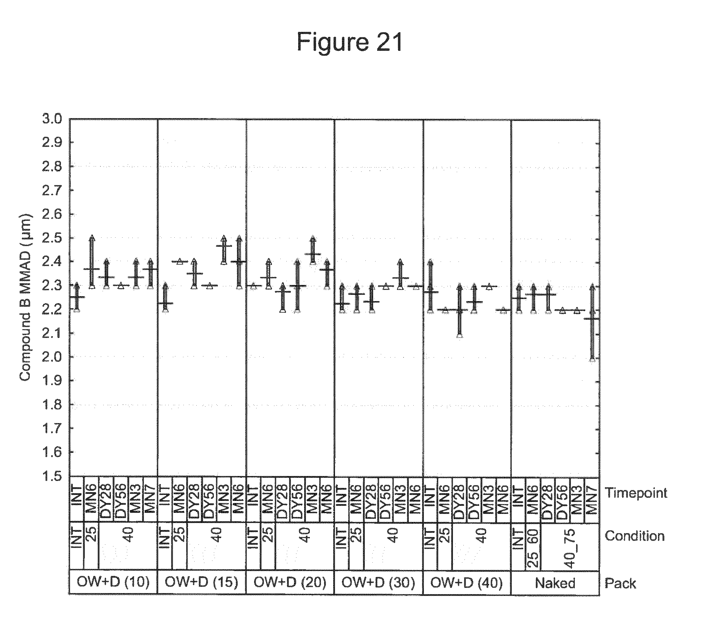

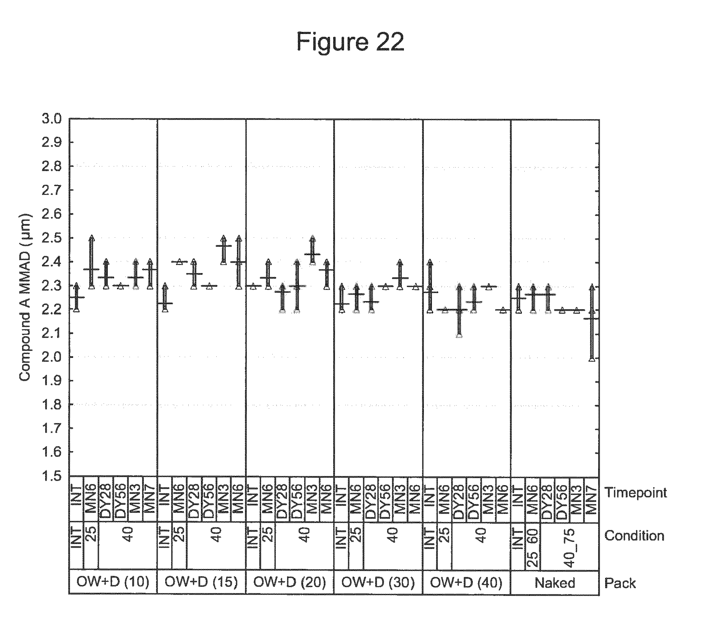

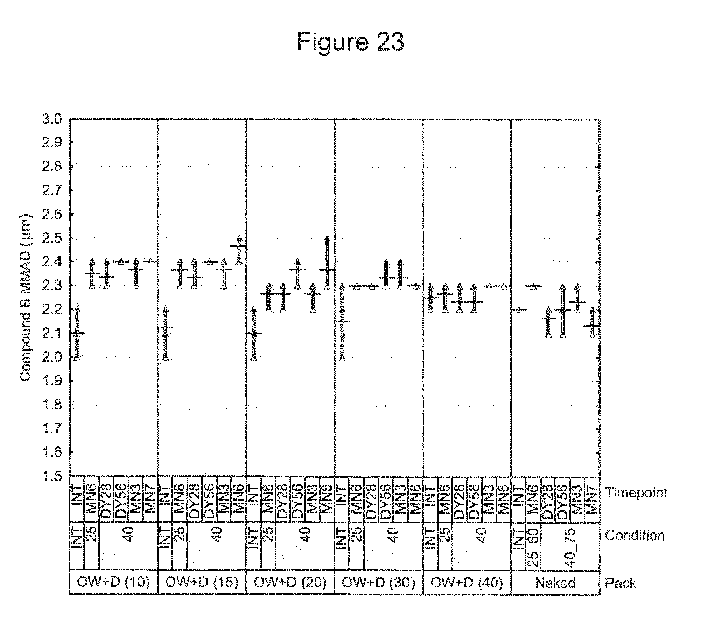

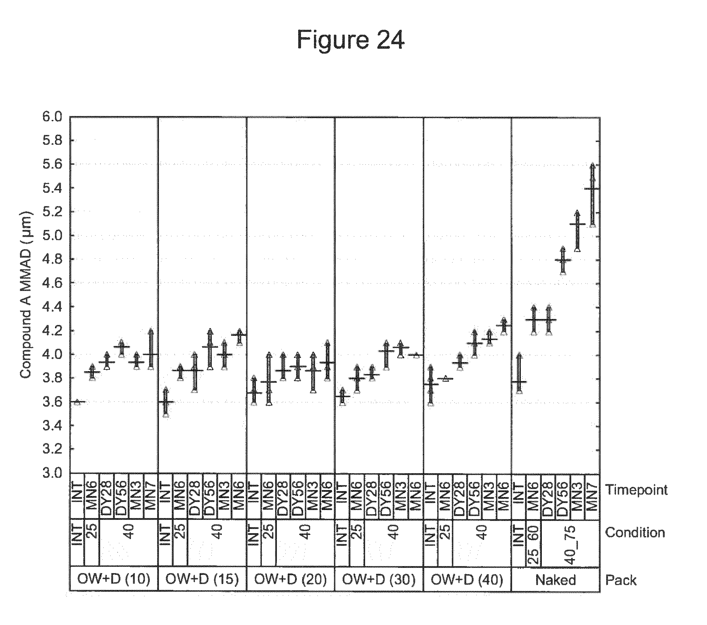

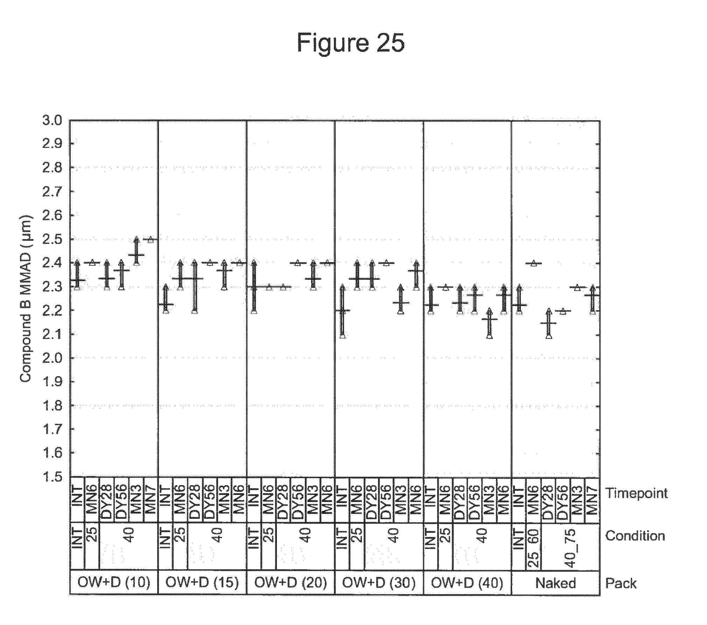

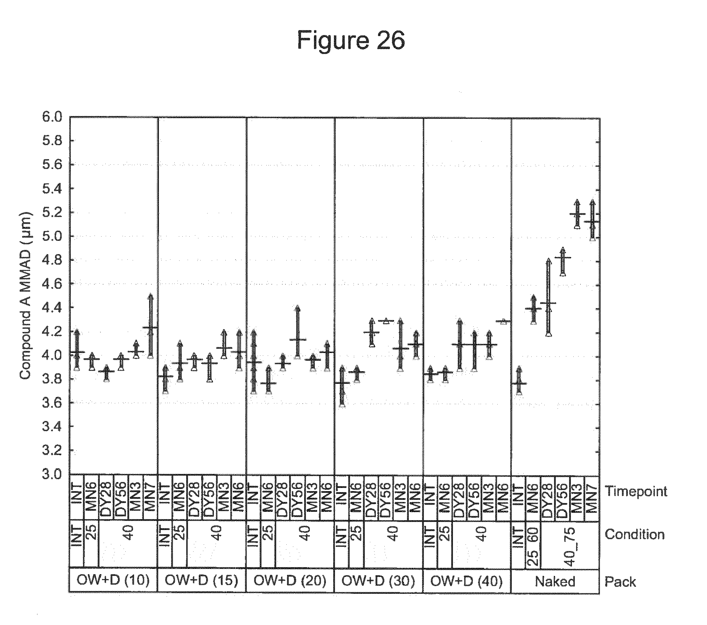

Figures 21-26 are illustrative of the changes in total for mass median aerodynamic diameter in microns as a function of both desiccant and storage conditions at select time intervals for the 50/25, 100/25, and 200/25 samples overwrapped with desiccant (OW+D) and unprotected. Figures 21, 23 and 25 show the changes in mass median aerodynamic diameter for blends 50/25, 100/25 and 200/25 of Compound B at 25°C/60% RH and 40°C/75% RH, respectively while Figures 22, 24 and 26 show the changes in mass median aerodynamic diameter for blends 50/25, 100/25 and 200/25 for Compound A at 25°C/60% and 40°C/75% RH respectively.

### Example 6

### Relationship of desiccant, humidity and temperature to inhalable formulation geometric standard deviation (GSD)

The comparison of product stored unprotected, packed in foil laminate 2 (overwrapped) and packed in foil laminate 2 with desiccant (overwrapped with desiccant) providing a range of relative humidities (RH) within the pack is illustrated herein as it relates to geometric standard deviation (GSD) as a function of storage conditions over time.

Blends were manufactured and filled using standard equipment. The inhalation device contained two packs in peelable blister strip form, the first containing blend with 12.5 µg of compound B, 1% magnesium stearate, and the balance to 12.5mg of inhalation grade lactose (lactose fines 4.5% < than 4.5 microns by dry dispersion) and the second peelable blister strip containing 50 µg of compound A and the balance to 12.5mg of inhalation grade (lactose fines 6.5% < than 4.5 microns by dry dispersion) lactose. Samples were left unprotected, overwrapped or overwrapped with a desiccant packet. Desiccant humidity values were 15% (8g Eq-pak^{®}, 10% RH plus 8g Eq-pak^{®}, 20% RH), 20% (8g Eq-pak^{®}, 20% RH) and 30% (8g Eq-pak^{®}, 30% RH). The RH inside the OW packs was around 45% RH. Overwrapped samples and samples overwrapped with desiccant were subsequently stored in standard stability chambers and storage conditions were 25°C and 60% RH and 40°C and 75% RH. Unprotected samples were stored in standard stability chambers and storage conditions were 25°C and 75% RH. Samples were analyzed for GSD initially and at time intervals up to 3 months for unprotected product stored at 25°C and 75%RH, up to 6 months for overwrapped and overwrapped with desiccant samples stored at 40°C and 75% RH and up to 9 or 12 months for overwrapped and overwrapped with desiccant samples stored at 25°C and 60% RH. GSD was determined using cascade impaction with HPLC coupled to UV/visible and fluorescence detection.

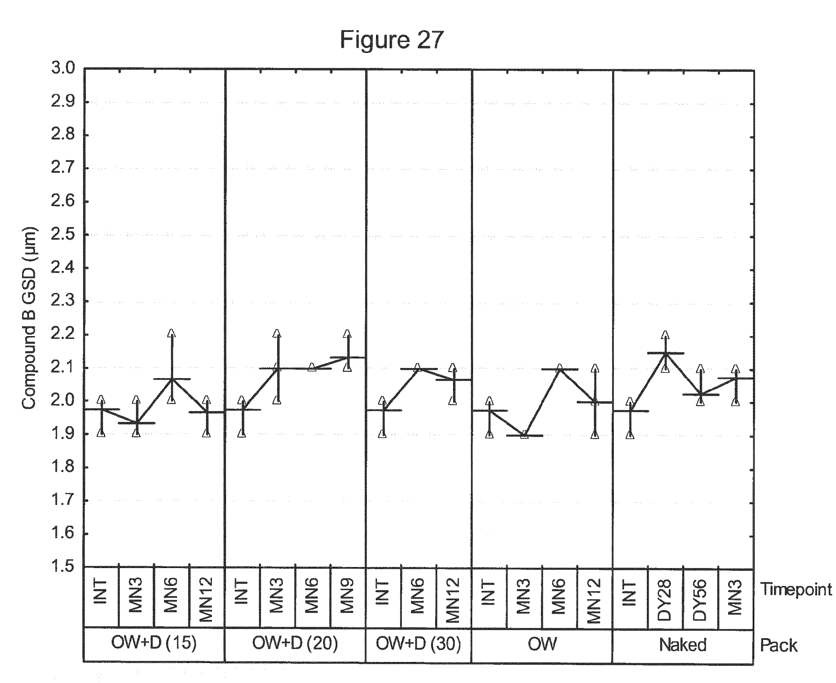

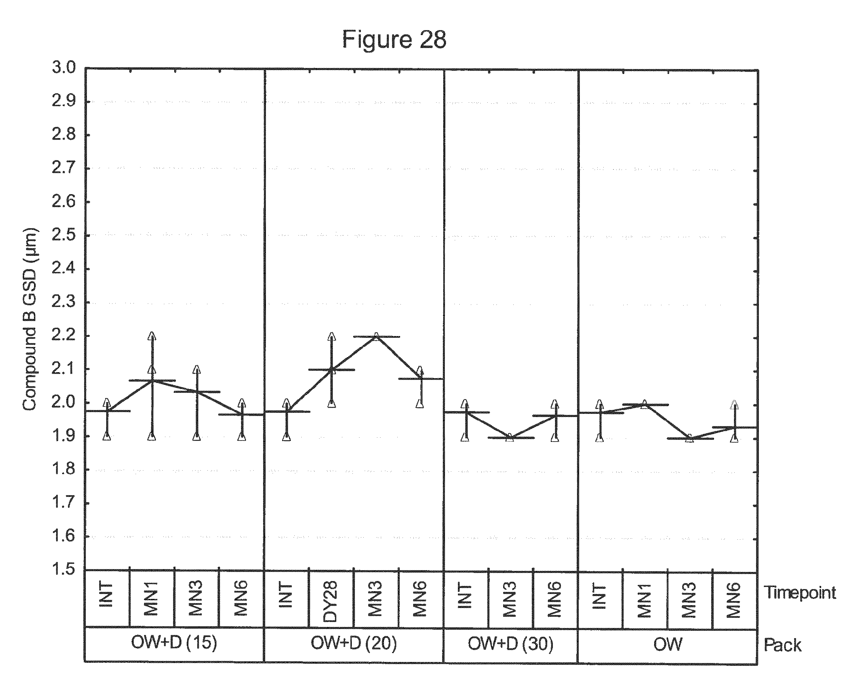

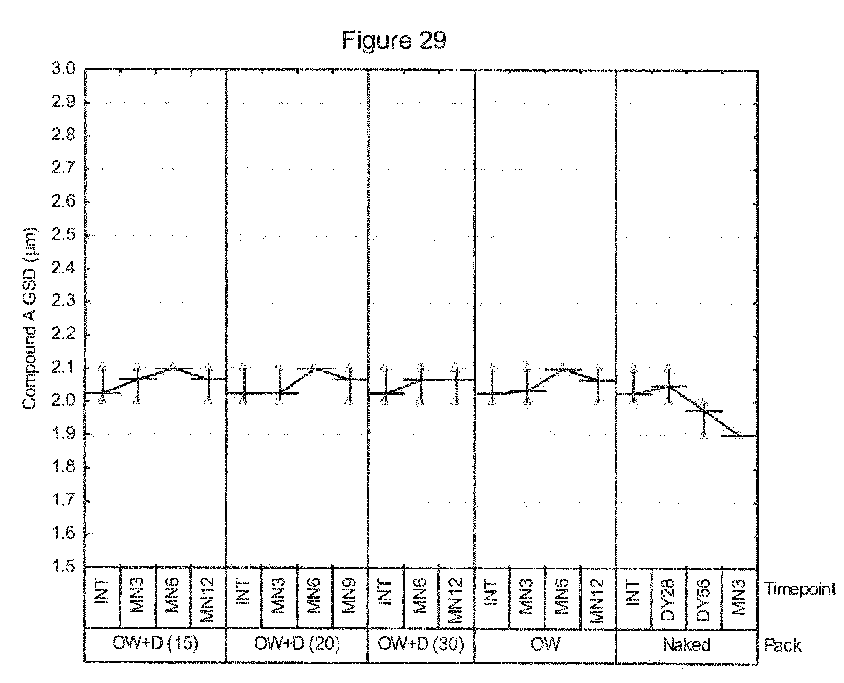

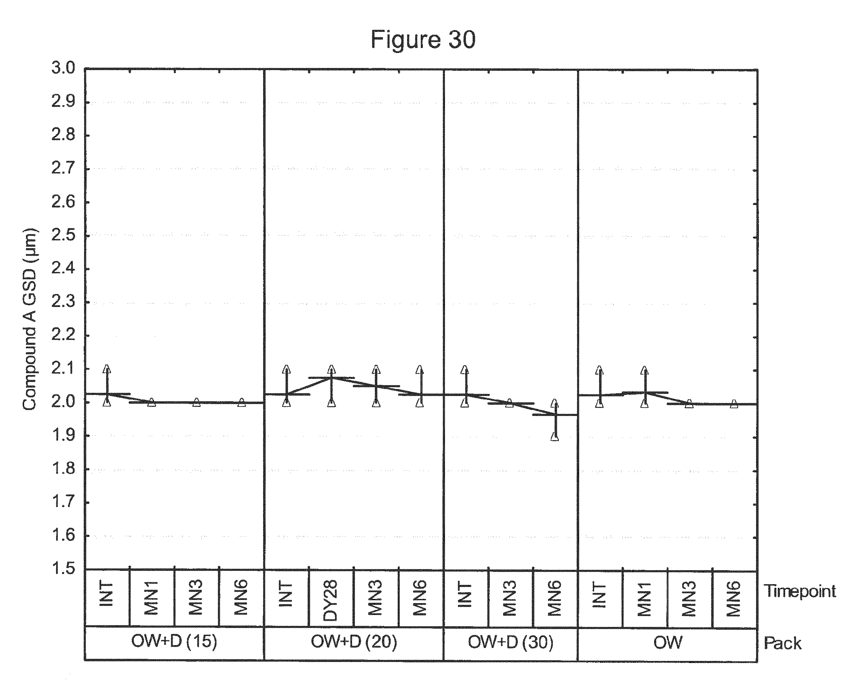

Figures 27-30 are illustrative of the changes in GSD, as a function of both desiccant RH values and storage time. Figures 27 and 29 show GSD values for samples stored overwrapped (OW) and overwrapped with desiccant (OW+D) at 25°C and unprotected at 25 °C and 75%RH at select time intervals for compound B and compound A respectively. Figures 28 and 30 show GSD values for samples stored overwrapped (OW) and overwrapped with desiccant (OW+D) at 40°C at select time intervals for compound B and compound A respectively.

### Example 7

### Relationship of desiccant, humidity and temperature to inhalable formulation geometric standard deviation (GSD)

The comparison of dual active formulated product, stored in foil laminate 1 with a desiccant (overwrapped with desiccant) or unprotected is illustrated herein as it relates to geometric standard deviation (GSD) as a function of storage time, relative humidity and temperature.

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7%, 6.2% and 5.7% < than 4.5 microns by dry dispersion respectively), 25µg of Compound B, 1% magnesium stearate, and the balance to 12.5mg of inhalation grade lactose (lactose fines 4.5% <4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet or left unprotected. Desiccant humidity values were 10% (8g Eq-pak^{®}, 10% RH), 15% (8g Eq-pak^{®}, 10% RH plus 8g Eq-pak^{®}, 20% RH), 20% (8g Eq-pak^{®}, 20% RH), 30% (8g Eq-pak^{®}, 30% RH) and 40% (8g Eq-pak^{®}, 40% RH). Overwrapped and Desiccated devices were subsequently stored in standard stability chambers alongside unprotected devices and storage conditions were 25°C and 60% RH and 40°C and 75% RH. Samples were analyzed for geometric standard deviation (GSD) at time intervals up to 6 months for samples stored at 25°C and 60% RH and up to 7 months for samples stored at 40°C and 75%. GSD was determined using cascade impaction with HPLC coupled to UV/visible and fluorescence detection.

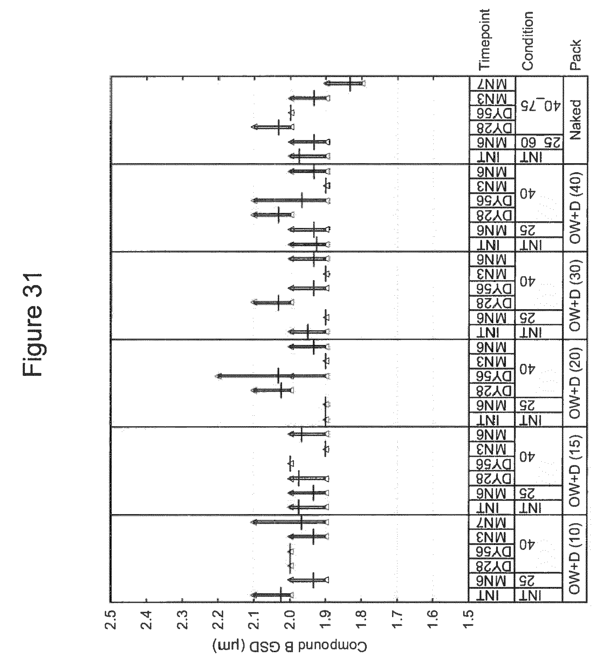

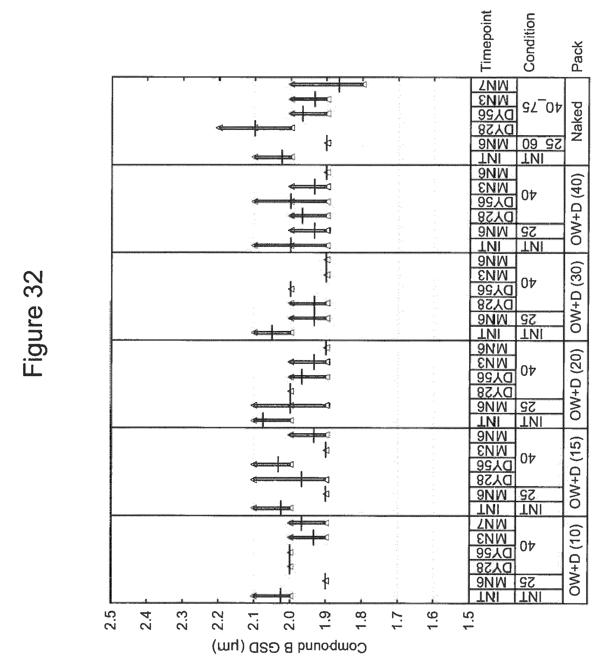

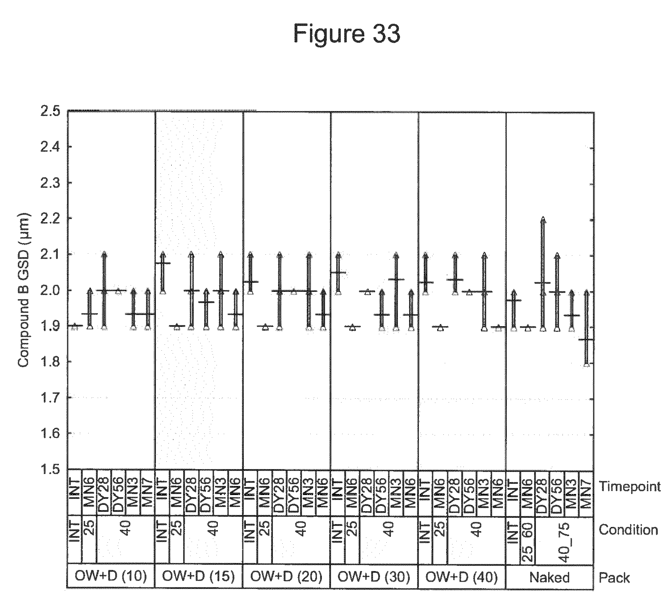

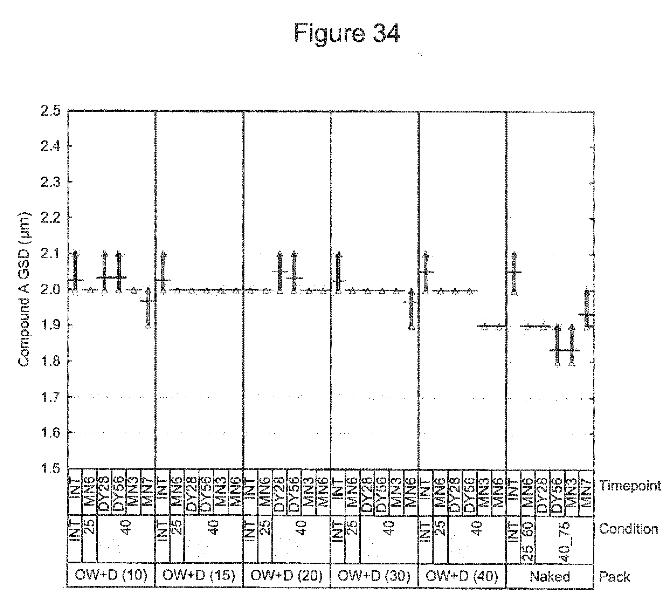

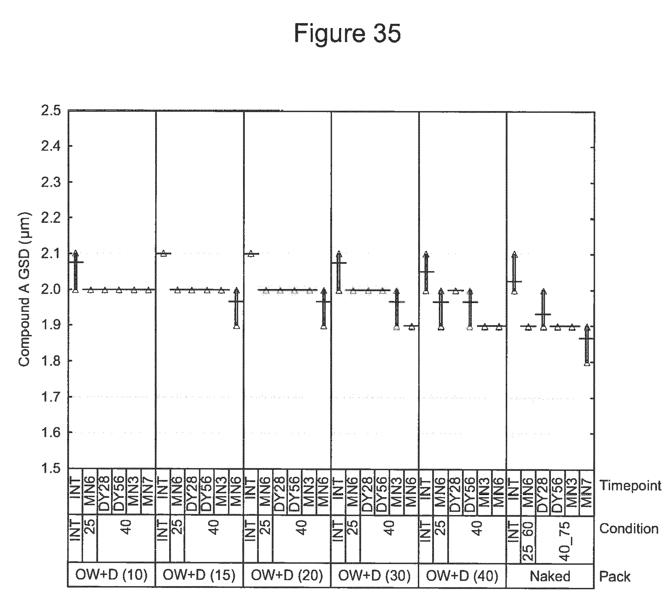

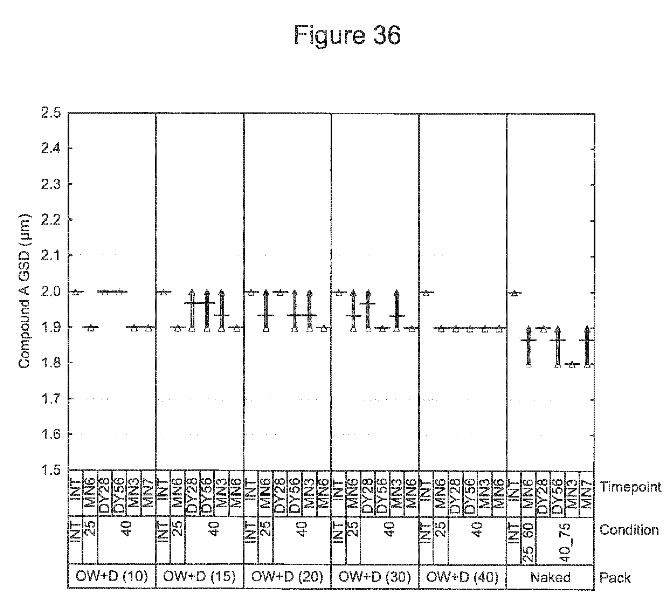

Figures 31-36 are illustrative of the changes in Geometric Standard Deviation as a function of both desiccant and storage conditions at select time intervals for the 50/25, 100/25, and 200/25 samples overwrapped with desiccant (OW+D) and unprotected. Figures 31, 32 and 33 show the changes in geometric standard deviation for blends 50/25, 100/25 and 200/25 of Compound B at 25°C/60% RH and 40°C/75% RH, respectively while Figures 34, 35 and 36 show the changes in geometric standard deviation for blends 50/25, 100/25 and 200/25 for Compound A at 25°C/60% and 40°C/75% RH respectively.

### Example 8

### Relationship of desiccant, humidity and temperature to Pack Relative Humidity (%)

The comparison of dual active formulated product, stored in foil laminate with a desiccant (overwrapped with desiccant) is illustrated herein as it relates to the pack relative humidity within the overwrap as a function of storage time, relative humidity and temperature.

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7 %, 6.2% & 5.7% < than 4.5 microns by dry dispersion respectively), 25µg of Compound B, 1% magnesium stearate, and the balance to 12.5mg of inhalation grade lactose (lactose fines 4.5% <4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet. Humidity values were 10% (8g Eq-pak^{®}, 10% RH), 15% (8g Eq-pak^{®}, 10% RH plus 8g Eq-pak^{®}, 20% RH), 20% (8g Eq-pak^{®}, 20% RH), 30% (8g Eq-pak^{®}, 30% RH) and 40% (8g Eq-pak^{®}, 40% RH). Overwrapped and desiccated devices were subsequently stored in standard stability chambers and storage conditions were 25°C and 60% RH and 40°C and 75% RH. Samples were analyzed for pack RH (%) at time intervals up to 6 months for samples stored at 25°C and 60% RH and up to 7 months for samples stored at 40°C and 75% RH. The Pack %RH was measured by inserting a pointed, narrow RH probe into the overwrap and recording the %RH when a stable value is attained (i.e., little or no variation), which as an example, typically occurs in two minutes. Typically a Rotronic Hygropalm meter with SC04 probe is used, although other measuring devices /techniques can be used.

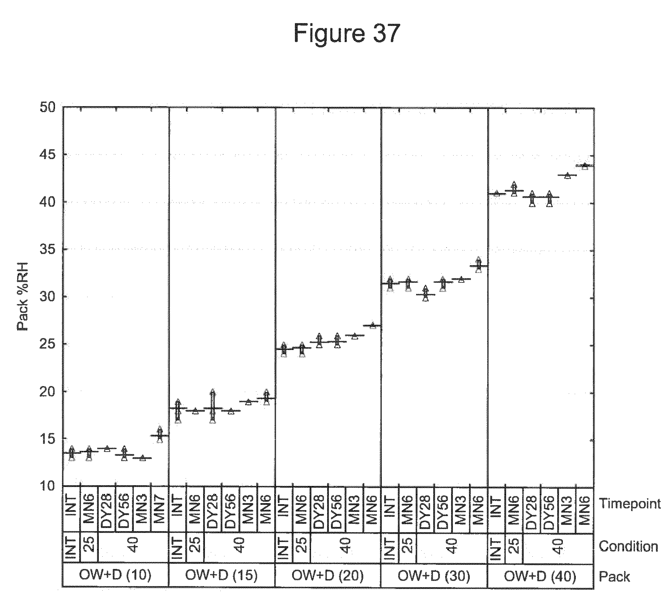

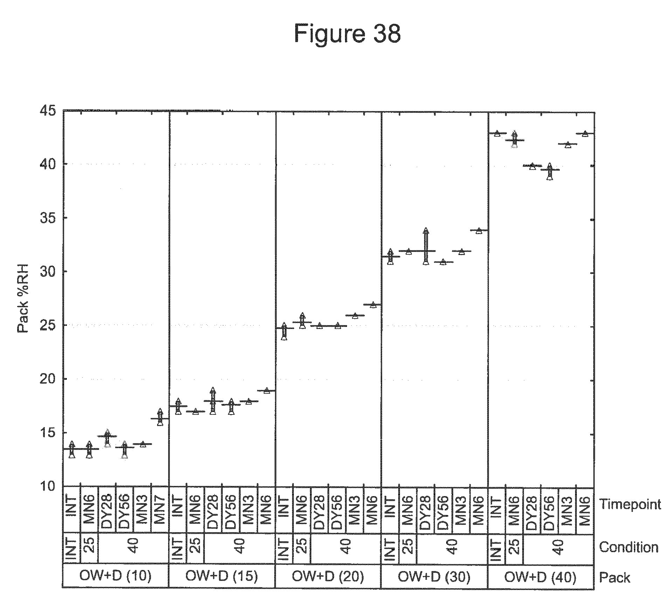

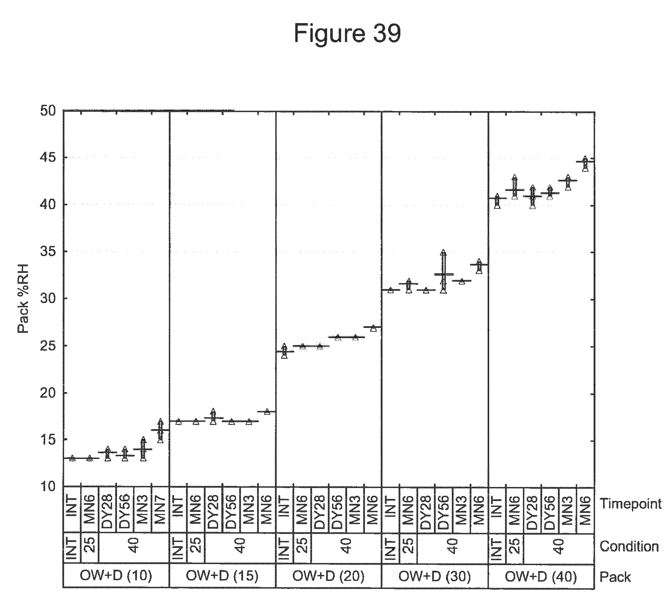

Figures 37-39 are illustrative of the Pack %RH in the overwrap as a function of both desiccant and storage conditions at select time intervals for the 50/25, 100/25, and 200/25 samples respectively overwrapped with desiccant (OW+D).

The application of which this description and claims form part may be used as a basis for priority in respect of any subsequent application. The claims of such subsequent application may be directed to any feature or combination of features described therein. They may take the form of product, method or use claims and may include, by way of example and without limitation, one or more of the following claims.

## Claims

1. A drug product comprising:
a dry powder inhalation device containing one or more pharmaceutical compositions present therein, wherein the one or more pharmaceutical compositions comprise active ingredients (I) 4-{(1*R*)-2-[(6-{2-[(2,6-dichlorobenzyl)oxy]ethoxy} hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof;
a hygroscopic material; and
a package which encompasses the dry powder inhalation device and the hygroscopic material defining an enclosed volume therein;
wherein each of the active ingredients (I) and (II) are present in the same or different pharmaceutical compositions, and wherein the hygroscopic material is in a state of partial hydration and has a moisture content range of from 8.4 to 18.6%.

2. A drug product according to claim 1 wherein the hygroscopic material has a moisture content range of from, at a lower end, any of the values, 8.4, 8.8, 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w to, at a higher end, any of the values 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w.

3. A drug product comprising:
a dry powder inhalation device containing one or more pharmaceutical compositions present therein, wherein the one or more pharmaceutical compositions comprise active ingredients (I) 4-{(1*R*)-2-[(6-{2-[(2,6-dichlorobenzyl)oxy]ethoxy} hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof;
a hygroscopic material; and
a package which encompasses the dry powder inhalation device and the hygroscopic material defining an enclosed volume therein;
wherein each of the active ingredients (I) and (II) are present in the same or different pharmaceutical compositions, and wherein the enclosed volume within the package exhibits a Relative Humidity of from, at a lower end, any of the values 15, 16, 17, 18 19, 20, 21, 22, 23, 24, 25, 26, 27, 28, 29, 30, 31, 32, 33, 34, 35, 36, 37, 38, 39 or 40 % RH to, at an upper end, any of the values, 17, 18, 19, 20, 21, 22, 23, 24, 25, 26, 27, 28, 29, 30, 31, 32, 33, 34, 35, 36, 37, 38, 39, or 40%RH.

4. A drug product according to claim 3 comprising:
a dry powder inhalation device containing one or more pharmaceutical compositions present therein, wherein the one or more pharmaceutical compositions comprise active ingredients (I) 4-{(1*R*)-2-[(6-{2-[(2,6-dichlorobenzyl)oxy]ethoxy} hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof;
a hygroscopic material; and
a package which encompasses the dry powder inhalation device and the hygroscopic material defining an enclosed volume therein;
wherein each of the active ingredients (I) and (II) are present in the same or different pharmaceutical compositions, and wherein the enclosed volume within the package exhibits a Relative Humidity of from 20% to 40%.

5. The drug product according to any of Claims 1 to 4, wherein the compound of (I) is 4-{(1*R*)-2-[(6-{2-[(2,6-dichlorobenzyl) oxy]ethoxy}hexyl)amino]-1-hydroxyethyl}-2-(hydroxymethyl)phenol triphenyl acetate and the compound of (II) is (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate.

6. The drug product according to any of Claims 1 to 5, wherein said one or more pharmaceutical compositions comprises at least one excipient.

7. The drug product according to Claim 6, wherein said at least one excipient is lactose.

8. The drug product according to any of Claims 1 to 7 wherein said hygroscopic material comprises silica gel.

9. The drug product according to Claim 8, wherein the silica gel is present within an enclosure.

10. The drug product according to Claim 9, wherein the silica gel is present within a packet.

11. The drug product according to any of Claims 1 to 10, wherein the packet is loose within the drug product.

12. The drug product according to any of Claims 10 or 11, wherein the packet is fixed with respect to the inhalation device.

13. The drug product according to any of Claims 1 to 12, wherein the dry powder inhalation device is a unit-dose device.

14. The drug product according to any of Claims 1 to 13, wherein the dry powder inhalation device is a multi-dose device.

15. The drug product according to claim 14 wherein said inhalation device permits separate containment of components.

16. The drug product according to claim 15 wherein said inhalation device is an inhaler device having two peelable blister strips, each strip containing pre-metered doses in blister pockets arranged along its length.

17. The drug product according to claim 16 wherein one blister strip has multiple blisters of composition containing the compound (I), and the other blister strip has multiple blisters of another composition containing the compound (II).

18. A drug product according to any of claims 1 to 17 for use in treating a respiratory disorder.

19. A process of producing a drug product comprising:
subjecting a hygroscopic material to moisture exposure sufficient to attain a predetermined relative humidity;
combining the hygroscopic material with an inhalation device containing one or more pharmaceutical compositions therein, wherein the one or more pharmaceutical compositions comprise active ingredients (I) 4-{(1*R*)-2-[(6-{2-[(2,6-dichlorobenzyl)oxy]ethoxy} hexyl)amino]-1-hydroxyethyl}-2-(hydroxymethyl)phenol, or a salt thereof, and (II) (6α,11ß,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; wherein each of the active ingredients (I) and (II) are present in the same or different pharmaceutical compositions, and
enclosing the dry powder inhalation device and hygroscopic material within a package to define an enclosed volume therein forming a drug product and wherein the hydration level of the hygroscopic material is such that enclosed volume has a Relative Humidity of from 20% to 40%.

20. A drug product produced according to the process of Claim 19.
